# EUROPEAN PATENT APPLICATION

(11) **EP 3 669 893 A1**
(43) Date of publication of application: **24.06.2020**
(21) Application number: 18382956.3
(22) Date of filing: 20.12.2018
(51) Int. Cl.: A61K 45/06, A61K 35/768, A61P 35/00

(54) **TREATMENT OF P53 MUTATED/MODIFIED CANCER-FORMS WITH PARVOVIRUS**

(71) Applicant: Universidad Autónoma de Madrid, 28049 Madrid (ES)
(72) Inventor: ALMENDRAL DEL RÍO, José María, 28049 Madrid (ES); GALLEGO, Carlos, 28049 Madrid (ES); GIL- RANEDO, Jon, 28049 Madrid (ES)
(74) Representative: Hoffmann Eitle

(57) **Abstract**

Treatment of cancer harboring mutations in the TP53 gene and/or post-translational modifications in the p53 protein. The present invention relates to the use of viruses belonging to the family *Parvoviridae*, genus *Protoparvovirus*, or combinations of such viruses with chemotherapy drugs, in the treatment of cancer. In particular the cancer is characterized by showing mutations in the TP53 gene and/or post-translational modifications in the p53 protein.

## Description

### FIELD OF THE INVENTION

The present invention is encompassed within the field of oncology. Particularly, the present invention refers to the use of viruses belonging to the *Parvoviridae* family, preferably to the genus *Protoparvovirus,* or to combinations of such viruses with chemotherapy, in the treatment of cancer. In particular, cancer is characterized by presenting mutations in the *TP53* gene and / or post-translational modifications in the p53 protein.

### STATE OF THE ART

### P53 protein and cancer

The p53 protein, which is expressed from the *TP53* gene, performs essential functions through very diverse mechanisms in the control of the cell cycle and genomic stability, which is why it has sometimes been called "the guardian of the genome". Therefore it is not surprising that mutations in the *TP53* gene be one of the main mechanisms responsible for induction of multiple and diverse types of cancers in humans. In fact, *TP53* is the most frequently mutated gene in human cancer and, in particular, various genetic changes in the gene *TP53* are frequently found in glioblastoma (GBM) (Brennan, CW et al (2013) The somatic genomic landscape of glioblastoma, Cell 155, 462-477), a devastating disease without effective treatment. The genetic alterations in *TP53* and in other genes cause the growth of GBM tumors to be governed by functionally redundant signalings, which allows their adaptation in responses to directed molecular therapies. It should therefore be emphasized that, despite the enormous importance of mutations in TP53 for the initiation and progression of multiple cancers being currently suffered by millions of humans, there is no specific effective treatment against these genetic lesions, as today TP53 is mainly considered as a "non-druggable" gene (Kastenhuber, ER, and S.W. Lowe. (2017), Putting p53 in context, Cell 170.1062-1076).

### Conventional cancer chemotherapy and p53

In the current oncology clinic, the chemotherapeutic regimes frequently use (alone or in combination) the following drugs:
- Hydroxyurea (HU or OH-U), which decreases the concentrations of dNTPs (nucleosides triphosphates) by inhibiting ribonucleotide reductase, and therefore stops the replication fork of the DNA (as does gemcitabine or cytosine arabinose), causing diverse cell responses that may include p53.
- Cisplatin (CisPt), is frequently used in the clinic in systemic treatments since more than forty years ago because its activity against diverse types of solid cancers *[*Basu A, Krishnamurthy S. 2010. Cellular responses to cisplatin induced DNA damage. J Nucleic Acids doi: 10.4061 / 2010/201367]*.*
- 5-fluorouracil (5FU), an antimetabolite analogous to uracil for general use against various cancers, although with greater efficacy against colorectal cancer. Its mechanism of action is complex and includes alterations of heterochromatin and RNA metabolism.

### Oncolytic viruses

Multiple viruses have demonstrated anti-cancer ability (oncolysis) in different systems, used as natural strains or genetically modified. In these regards, it should be highlighted at least the following types of viruses with some demonstrated oncolytic capacity: parvovirus, measles virus, reovirus, adenovirus, herpesvirus and poxvirus. However, it has not been identified in the state of the art oncolytic viruses selectively acting against cancers harboring TP53 mutations and/or post-translational modifications in the p53 protein. In other words, the ability of oncolytic virus has not been specifically linked so far to mutations in the *TP53* gene and/or post-translational modifications in the p53 protein.

The present invention therefore focuses on solving the technical problem explained above, by identifying oncolytic virus for use alone or in combination with chemotherapy, to especially target cancers with mutations in the *TP53* gene and/or post-translational modifications in the p53 protein. The present invention thus provides an effective therapeutic window, allowing specific and custom treatments against tumors harboring genetic alterations in the *TP53* gene and/or post-translational modifications in the p53 protein, such as those often found in human primary cancers.

### DESCRIPTION OF THE INVENTION

### Brief description

The present invention refers to the use of viruses belonging to the *Parvoviridae* family, particularly to the *Protoparvovirus* genus, or combinations of such viruses with chemotherapy in the treatment of cancer. Noteworthy, many types of Cancers are characterized by presenting mutations in the *TP53* gene and/or post-translational modifications in the p53 protein. Furthermore, in a preferred aspect, the present invention demonstrates that such viruses cooperate synergistically in their toxic effects against cancer cells with conventional chemotherapy, provided that the target cells harbor genetic alterations in the gene *TP53* gene and/or post-translational modifications in the p53 protein, either constitutive or induced by these drugs.

Therefore, the present invention breaks a prejudice in the state of the art, because it is generally assumed a good correlation between the presence of TP53 mutations in cancer patients and the adverse outcome of chemo- and radiotherapy treatments, although the spectrum of mutations involved in each case is yet to be defined [Tchelebi, L., Ashamalla, H., and Graves PR (2014) Mutant p53 and the response to chemotherapy and radiation. In: Deb S., Deb S. (eds) Mutant p53 and MDM2 in Cancer. Subcellular Biochemistry, vol 85. Springer, Dordrecht]. In contrast, the present invention demonstrates an effective treatment of various types of cancers characterized by presenting mutations in the *TP53* gene and/or a post-translational modification in the p53 protein, when the parvoviruses of the invention are used, achieving a synergistic effect in combination with genotoxic chemotherapeutic agents.

Thus, the present invention, through the figures and examples set forth below, demonstrates that:
- Several strains of viruses of the *Parvoviridae* family, particularly of the genus *Protoparvovirus* tested in the present invention, selectively infect in a toxic manner (by expressing the viral NS1 protein) human cancer cells of very different origins, including primary stem cells from glioblastoma patients, provided they harbor mutations in the *TP53* gene.
- Non-physiological post-translational modifications of the p53 protein, particularly constitutive or induced phosphorylation in the residue Serine 15, favor the toxic infection and replication in diverse human cancer cells of the virus strains tested in the present invention, including primary stem cells of patients with glioblastoma.
- Alterations induced in the p53 protein by oncogenic virus proteins, either present in primary tumors or expressed exogenously in stem cells and established cell lines of human glioblastoma, do facilitate the toxic infection and replication of the *Parvoviridae* virus strains tested in the present invention.
- Chemotherapeutic drugs of current clinical cancer therapies (such as OH-U, 5FU or Cisplatin) which induce post-translational modifications in the p53 protein (for example phosphorylation in the residue Ser15), act synergistically with the toxic infection of the *Parvoviridae* virus strains tested in the present invention, decreasing very significantly the viability of these cancer cells.

Therefore, the first aspect of the present invention relates to a viral particle comprising a nucleotide sequence consisting essentially of: SEQ ID NO: 1, SEQ ID NO: 2, SEQ ID NO: 3 or SEQ ID NO: 4, for use, alone or in combination with genotoxic chemotherapy drugs, in the treatment of cancer, where the cancer is characterized by presenting mutations in the TP53 gene and/or post-translational modification(s) in the p53 protein. Furthermore, viral particles with at least 95%, at least 96%, at least 97%, at least 98% or at least 99% sequence identity with the sequences SEQ ID NO 1, SEQ ID NO: 2, SEQ ID NO: 3 or SEQ ID NO: 4 are included in the present invention.

It is important to highlight the high percentage of homology among the four viral sequences belonging to the genus *Protoparvovirus* of the *Parvoviridae* family included in the present invention, as shown in **Table 1.** It is noteworthy to further note that the differences in nucleotides are placed mainly in non-coding regions of the genomes, so if we stick to the coding regions the percentage of homology would be even higher.

**TABLE 1**

| **% Identity** | **MVMp SEQ ID NO: 1** | **MVMi SEQ ID NO: 2** | **LuIII SEQ ID NO: 3** | **H-1 SEQ ID NO: 4** |
|---|---|---|---|---|
| **MVMp SEQ ID NO: 1** | **100** | **97** | **84** | **86** |
| **MVM i SEQ ID NO: 2** | 97 | **100** | **84** | **85** |
| **LuIII SEQ ID NO: 3** | 84 | 84 | **100** | **82** |
| **H-1 SEQ ID NO: 4** | 86 | 85 | 82 | **100** |

The second aspect of the present invention refers to a pharmaceutical composition comprising a viral particle which in turn comprises a nucleotide sequence consisting essentially of: SEQ ID NO: 1, SEQ ID NO: 2, SEQ ID NO: 3 or SEQ ID NO: 4, in combination with a genotoxic chemotherapy drug.

In a preferred aspect of the invention, post-translational modifications in the p53 protein are constitutive, induced by chemotherapy drugs that induce damage to ADN or genotoxic stress [Kirkland, D. et al. Updated recommended lists of genotoxic and non-genotoxic chemicals for assessment of the performance of new or improved genotoxicity tests. Mutation Research 795 (2016) 7-30] [Zhu, Y. et al. Cisplatin causes cell death via TAB1 regulation of p53 / MDM2 / MDMX circuitry. (2013). Genes and Develop 27: 1739-1751], or induced by oncogenic viruses. In a preferred aspect of the invention, the post-translational modification of the p53 protein consists in the phosphorylation of the Ser15 residue. In a preferred aspect of the invention, the *TP53* gene mutation is selected from the group comprising: R273H, P72R, E258K, G245S and/or V173L. In a preferred aspect of the invention, the *TP53* gene mutation is found in the DNA-binding domain (DBD) of the encoded p53 protein. In a preferred aspect of the invention, the genotoxic chemotherapy drug is selected from the group comprising: cisplatin, hydroxyurea, 5-fluoruracyl, gemcitabine, or cytosine arabinoside.

In a preferred aspect of the invention the cancer is glioma, lung cancer, esophageal cancer, liver cancer, pancreatic cancer, bladder cancer, colorectal cancer, prostate cancer, glioblastoma, glioma, head and neck cancer, cancer of the breast, stomach cancer, ovarian cancer, uterine cancer or melanoma. In a preferred aspect of the invention the said viral particle is used in combination with a genotoxic chemotherapy drug, where the viral particle is administered at the same time, after, or before the genotoxic chemotherapy drug.

The third aspect of the present invention refers to the *in vitro* method for the determination of mutations in the TP53 gene and/or post-translational modifications in the p53 protein comprising the use of a viral particle comprising a nucleotide sequence consisting essentially of: SEQ ID NO: 1, SEQ ID NO: 2, SEQ ID NO: 3, or SEQ ID NO: 4.

The fourth aspect of the present invention relates to a method for the *in vitro* diagnosis of cancer, or for selection of cancer patients comprising the determination of mutations in the *TP53* gene and/or post-translational modifications in the p53 protein by using the viral particle comprising a nucleotide sequence consisting essentially of: SEQ ID NO: 1, SEQ ID NO: 2, SEQ ID NO: 3, or SEQ ID NO: 4.

The fifth aspect of the present invention relates to mutations in the TP53 gene and/or post-translational modifications in the p53 protein for use in the treatment of cancer, where the mutation of TP53 is selected from the group comprising: R273H, P72R, E258K, G245S or V173 L, and the post-translational modification of the p53 protein is phosphorylation at the Ser15 residue.

The sixth aspect of the present invention refers to a method for the treatment of cancer, characterized by presenting mutations in the TP53 gene and/or post-translational modifications in the p53 protein, comprising the administration of a therapeutically effective amount of a viral particle comprising a nucleotide sequence consisting essentially of: SEQ ID NO: 1, SEQ ID NO: 2, SEQ ID NO: 3 or SEQ ID NO: 4, and/or chemotherapy.

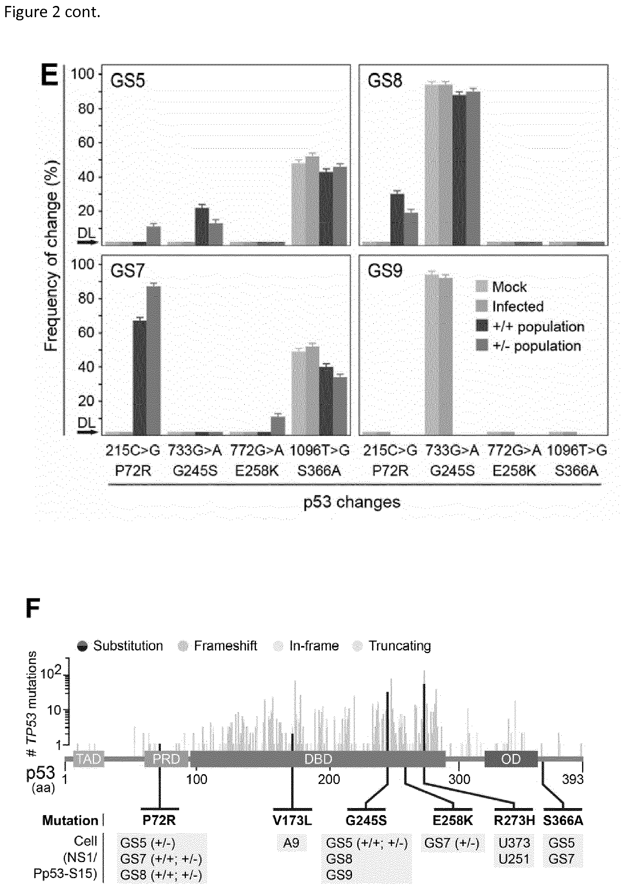

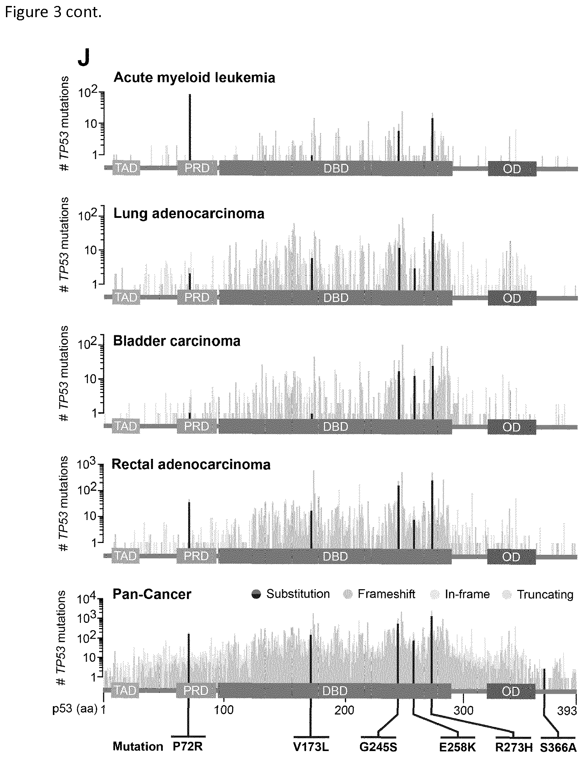

In a preferred aspect of the present invention the mutation of the *TP53* gene is selected from the group comprising: R273H, P72R, E258K, G245S and /or V173 L. In a preferred aspect of the present invention the mutation of the *TP53* gene is found in the DBD region or in the proline-rich domain (PRD) of the encoded p53 protein. In this regard, it is important to keep in mind that most mutations, and many of the more important mutations that occur in the *TP53* gene, are present in the domain DBD gene, and also that this fact may be considered reproducible in different types of cancers, as seen in **Figure 3J****.** This means that the utility of the present invention could be extrapolated to the treatment of any type of cancer with mutations in the *TP53* gene, particularly in the DBD domain, such as: lung cancer, esophageal cancer, liver cancer, pancreatic cancer, bladder cancer, colorectal cancer, prostate cancer, glioblastoma, glioma, cancer of the head and neck, breast cancer, stomach cancer, ovarian cancer, uterine cancer, some types of leukemia, or melanoma, among others. In fact, as significant example, the mutations R273H and G245S which are among the most frequently found not only in glioblastoma (**Figure 2F**) but also in different types of cancers, localize in the DBD domain of the *TP53* gene (**Figure 3J**). In a preferred aspect of the present invention the mutation is selected from the group comprising: R273H, E258K, G245S and /or V173 L. In a preferred aspect of the present invention the post-translational modification in the p53 protein consists of phosphorylation of the Ser15 residue. In a preferred aspect of the present invention the above mentioned TP53 mutations are isolated individually, or in combinations of at least two, at least three, at least four, at least five, or at least six of any of these mutations.

Preferably, in the said pharmaceutical composition or medicament, the viral particle is in a concentration of between 10⁶ to 10¹² pfu/ml (pfu: plaque forming unit), more preferably between 10⁷ and 10¹¹ pfu/ml, even more preferably between 10⁸ and 10¹⁰ pfu/ml.

This concentration of viral particles in the pharmaceutical composition can be referred, within the framework of the invention, to the concentration of a single type of viral particle, the pharmaceutical composition not containing any other type of viral particle. Alternatively, the indicated concentration can be achieved by mixtures of various types of viral particles as defined above, together reaching the indicated concentration. All possible combinations that will look apparent to one skilled in the art are included within the scope of the present invention.

The pfu/ml is a quantitative measure usually used in virology, and corresponds to the number of infectious viral particles capable of forming lysis plaques in monolayers of susceptible cells per volumetric unit. It is a functional measure rather than a measure for the absolute number of particles: virus particles that are defective or that fail to infect their target cells will not produce a plaque, and therefore will not be counted. For example, a composition comprising parvovirus MVM in a concentration of 10⁶ pfu/ml indicates that 1 milliliter of the composition contains enough virus particles to produce 10⁶ lysis plaques in a cell monolayer, but it is not possible to establish a relationship between pfu and the actual number of physical virus particles by this assay. By complementary methods, for example by agglutination of red cells or by electron microscopy, it is possible to determine the total number of viral particles in a preparation whether or not infectious.

According to a preferred embodiment, the pharmaceutical composition comprises at least one pharmaceutically acceptable carrier.

According to a preferred embodiment, the pharmaceutical composition comprises at least one pharmaceutically acceptable excipient

According to another preferred embodiment, the pharmaceutical composition comprises at least one pharmaceutically acceptable adjuvant.

Preferably, in the pharmaceutical composition the vehicle or excipient is such as to allow administration of said composition intratumorally (in solid tumors), intracerebrally, intraperitoneally, intravenously, intramuscularly, subcutaneously, intracutaneously, intracecally (or intrathecally), intraventricularly, orally, enterally, parenteral, intranasal or dermal. More preferably, the administration is intracerebrally, intravenously, or intranasally.

For the purposes of the present invention, the following definitions are provided:
- The term "comprising" means that it includes, but is not limited to, what follows the word "comprising". Therefore, the use of the term "comprising" indicates that the elements listed are mandatory, but that other elements are optional and may or may not be present.
- By "consisting of' is meant to include, and is limited to, what follows the phrase "consisting of'. Therefore, the phrase "consisting of' indicates that the elements listed are mandatory, and that other elements cannot be present.
- "Pharmaceutically acceptable excipient" refers to an excipient that can optionally be included in the compositions of the invention, and that it does not cause significant adverse toxicological effects to the patient.
- By "therapeutically effective dose or amount" of the composition of the invention it is understood a dose that when administered causes a positive therapeutic response in a subject suffering from cancer. The exact amount required will vary from one subject to another, depending on the age, the general conditions of the subject, the severity of the disease being treated, the mode of administration, and other related factors. The appropriate "effective" amount can be determined by a person skilled in the art using routine experimentation, based on the information provided in the state of the art.

### Description of the figures

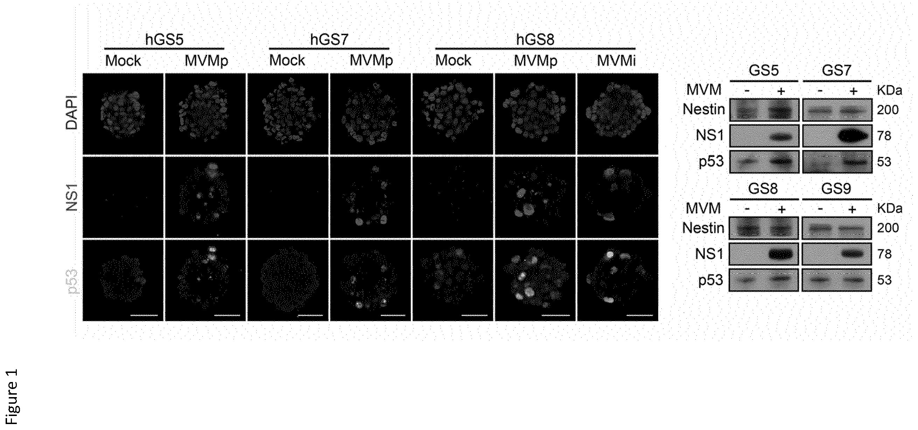

**Figure 1****.** The parvovirus minute virus of mice (MVM) induces p53 in human glioblastoma stem cells (GS, or hGS). Stem cells obtained from glioblastoma patients were infected with the MVMp (prototypic strain) and MVMi (immunosuppressive strain) viruses and analyzed for p53 protein expression. *Left:* analysis by immunofluorescence (IF) in confocal microscopy for the expression of the viral protein NS1 and the cellular p53 in GS cells of patients # 5,7 and 8 growing as neurospheres. Mock, uninfected cells. *Right:* analysis of the induction of the same proteins by western-blot in GSs of the four patients. Nestin: loading control. The molecular masses (kDa) are indicated on the right.

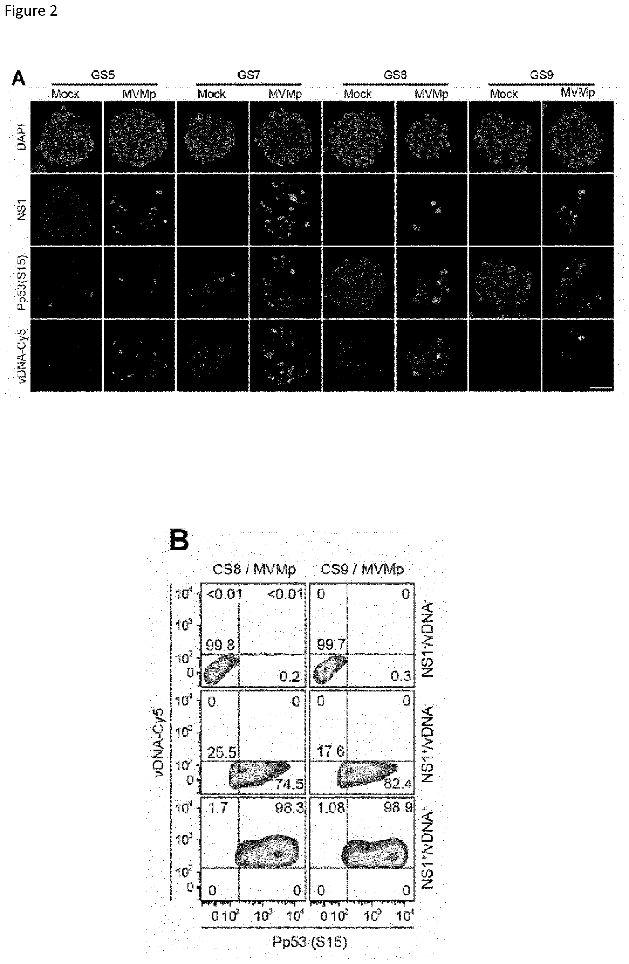

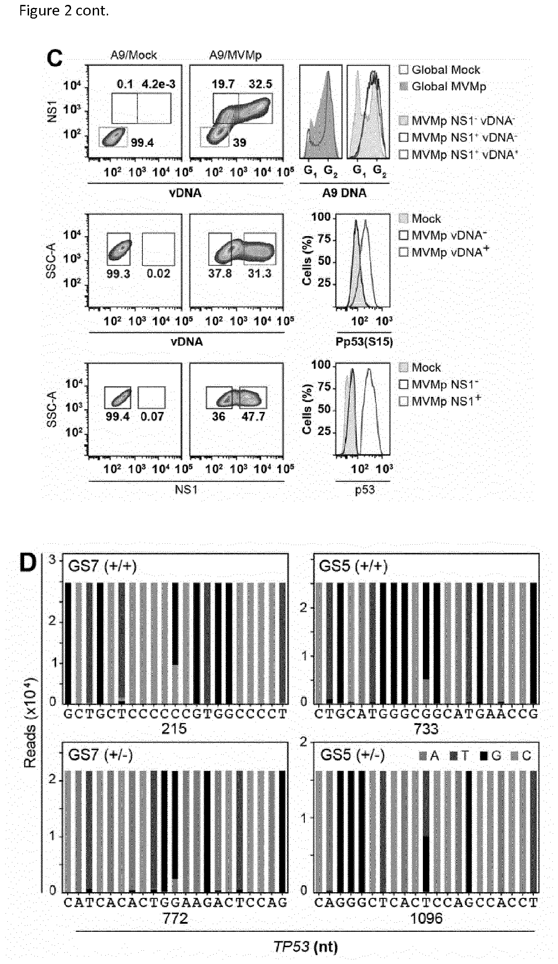

**Figure 2****.** Post-translational modification of p53 and genetic alterations of TP53 in GS cells permissive to the cytotoxic infection and replication of the parvovirus MVM. A. Analysis by IF in confocal microscopy of the correlation between the expression of NS1, the induction of p53 phosphorylated in Ser15, and the replication of the MVMp genome, in glioblastoma stem cells (GS) from four patients (# 5,7, 8, 9). **B.** Cytometric analysis, in GS of two patients, of the correlation between the level of MVMp genome replication and the phosphorylation of p53 in Ser15. **C.** Cytometry of A9 mouse fibroblasts infected by MVMp and analyzed for correlation between the levels of: NS1 expression and virus DNA replication (upper), modification of p53 in S15 and virus DNA replication (medium), and NS1 expression and p53 induction (bottom). **D.** Illustration of some mutations in TP53 detected by massive sequencing (NGS) in GS of two patients previously infected and drawn by the expression of NS1 (+) / p53-S15 (+). **E.** Frequency of appearance of TP53 mutations detected by NGS in GS of three patients infected and drawn by the expression of NS1(+)/p53-S15 (+). **F.** Representation of the four mutations detected by NGS in the p53 protein domains in GS cells of four patients infected with MVMp and drawn by expression of NS1 (+) / p53-S15 (+). The positions of the V173L (present in mouse A9 fibroblasts as V170L) and R273H (present in the U373MG and U251MG human glioblastoma cell lines) are also indicated. The type and number of accumulated absolute cases of all described mutations in the TP53 domains (TAD, transactivation domain; PRD, proline- rich domain; DBD, DNA-binding domain; OD, oligomerization domain) as reported by the consortiums TCGA (the Cancer Genome Atlas, NIH USA; https://portal.gdc.cancer.gov*)* and Cosmic (Sanger Institute of the UK; https://cancer.sanser.ac.uk/cosmic) in human GBM samples are illustrated.

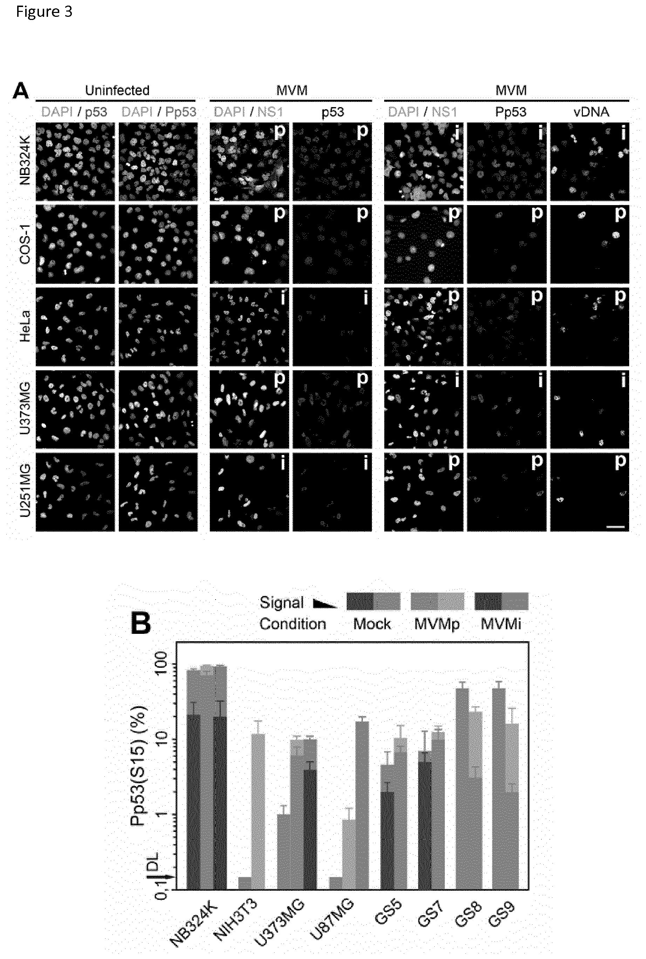

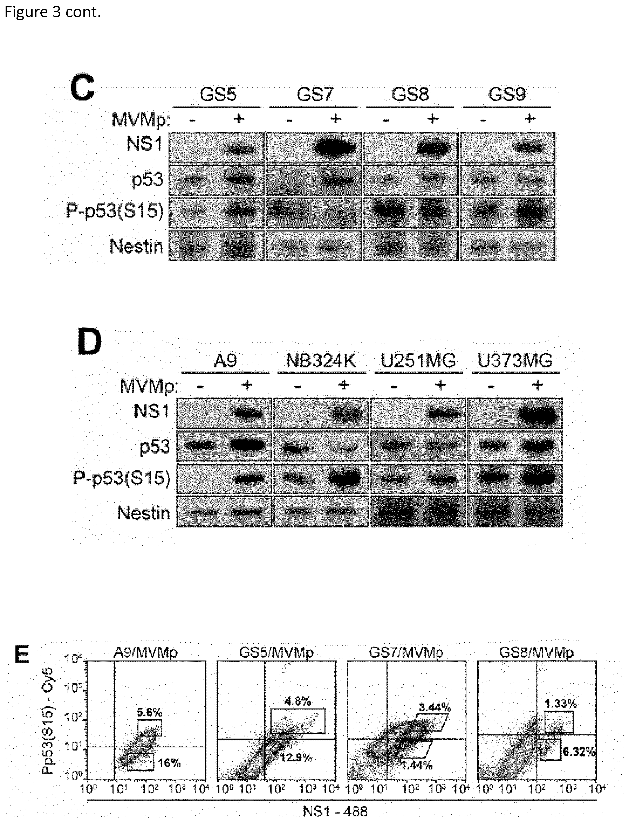

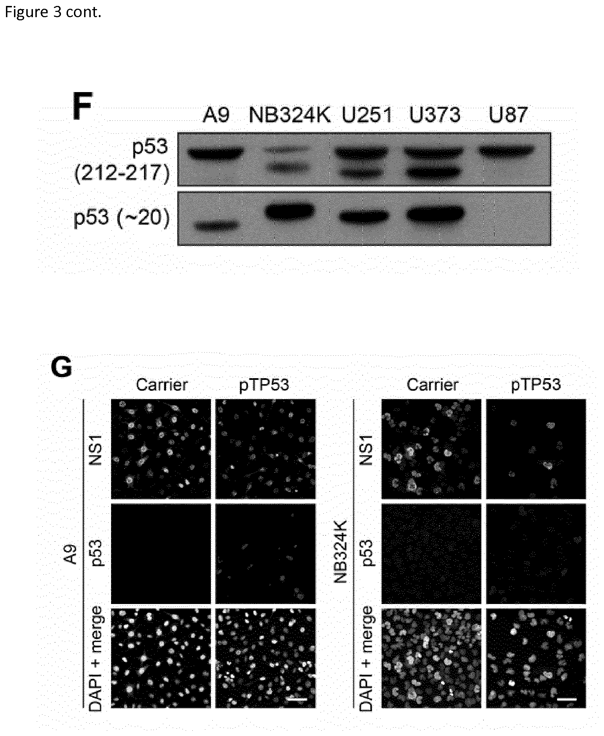

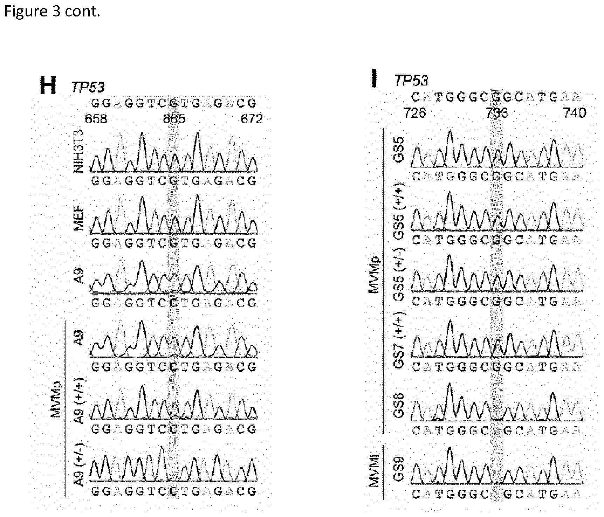

**Figure 3****.** Post-translational modification in p53 and genetic alterations of TP53 in established cell lines of human cancer and GS infected with the parvovirus MVM strains. **A.** Confocal IF panels of established human and primate cell lines infected with two strains (i, p) of parvovirus MVM and stained for p53, p53 phosphorylated at Ser15 (pp53), and parvovirus DNA (vDNA). **B.** Confocal IF estimation of the p53-S15 frequency and expression level in different uninfected (mock) or infected cells with the MVM strains. **C, D.** Western blot analysis of the induction of NS1, p53 and p53-S15 in GS (C) and different established lines of mouse (A9) and human glioblastoma (D). **E.** Purification by sorting of A9 and GS cells based on the expression of NS1 + and p53-S15 +. The indicated populations were used for the genetic analysis of TP53. The percentage of cells positive for the markers in the selected windows is indicated. **F.** Western blot analysis of p53 recognition by specific antibodies against two regions of the protein in different cell lines. Note the lack of recognition of p53 in the U87MG cell line by the antibody specific for the approx-20 domain. **G.** wtTP53 prevents the expression of the virus NS1 protein. Confocal IF analysis of the expression of p53 and NS1 in A9 and NB324K cells transfected with a plasmid expressing wtp53 or with carrier DNA. **H, I.** Chromatograms showing Sanger sequencing of p53 regions in the established cells and indicated pooled populations (H) or in GS (I). **J.** Representation of the TP53 mutations detected in this study in relation to all those described in the TP53 domains (TAD, transactivation domain, PRD, proline-rich domain, DBD, DNA-binding domain, OD, oligomerization domain) by the TCGA (The Cancer Genome Atlas, NIH USA; https://portal.gdc.cancer.gov*)* and Cosmic (Sanger Institute of the UK ; https://cancer.sanger.ac.uk/cosmic) consortium in the indicated types of human cancers. The scheme shows how most mutations in different types of cancer occur in the DBD domain of the TP53 gene, although the frequency of a particular mutation can be very high in a certain type of cancer.

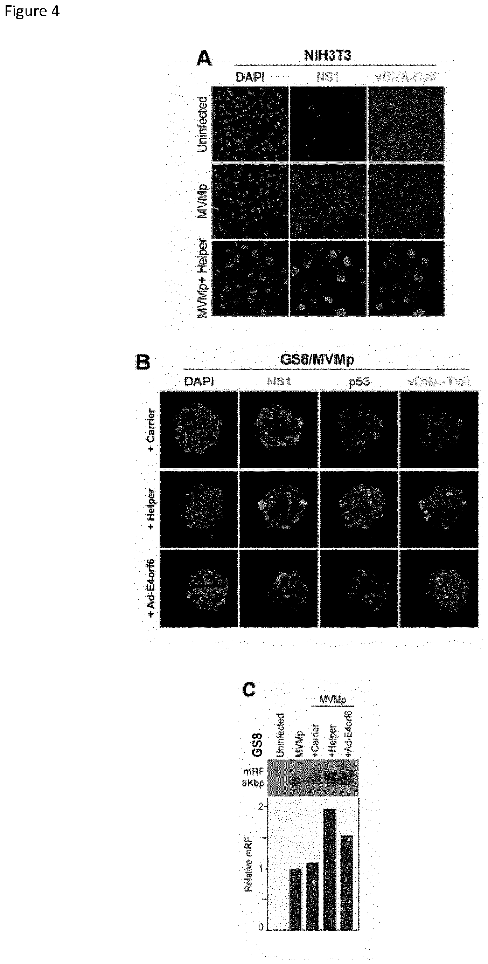

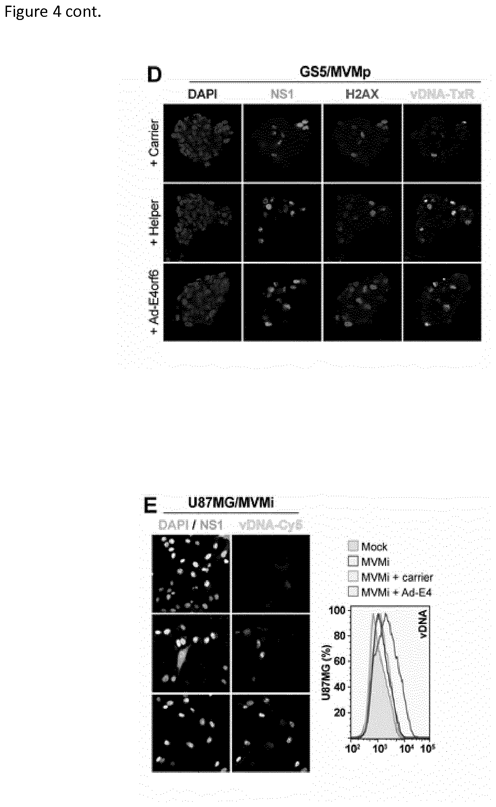

**Figure 4****.** Parvovirus MVMp and MVMi infections of mouse and human cell lines, and GS of patients, transfected with adenovirus oncogenes that alter p53. **A.** Confocal IF analysis of NIH3T3 cells, or (**B**) GS8 cells, transfected with plasmids expressing oncogenes showing increased replication of the MVMp genome. **C.** Similar study in GS8 cells showing accumulation levels of the main replicative intermediate (mRF) of the MVM genome analyzed by southern-blot. **D.** Effect of transfection with plasmids expressing oncogenes on the replication of the MVMp genome in GS8 cells, or (**E**) of the MVMi genome in U87MG measured by IF-confocal (left) or cytometry (right).

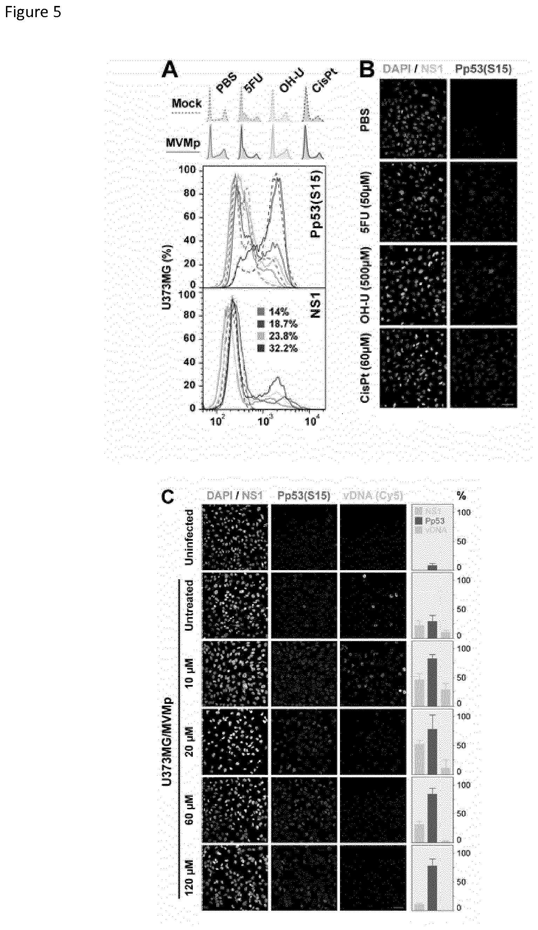

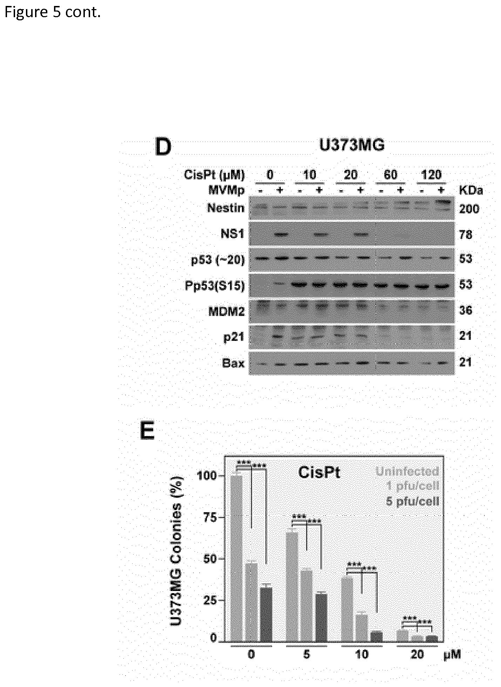

**Figure 5****.** Effects of chemotherapy drugs (CD) on the infection of U373MG glioblastoma cells with parvovirus MVMp. **A.** Analysis by cytometry and by confocal IF (**B**) of the effect of three CD on the levels of NS1 and p53-S15 expression. **C.** IF-confocal analysis of dose effect of Cisplatin in the levels of NS1, p53-S15, and virus genome replication. **D.** Analysis under similar conditions measuring levels of NS1 and p53 signaling proteins by SDS-blot, or (**E**) cell viability by colony formation.

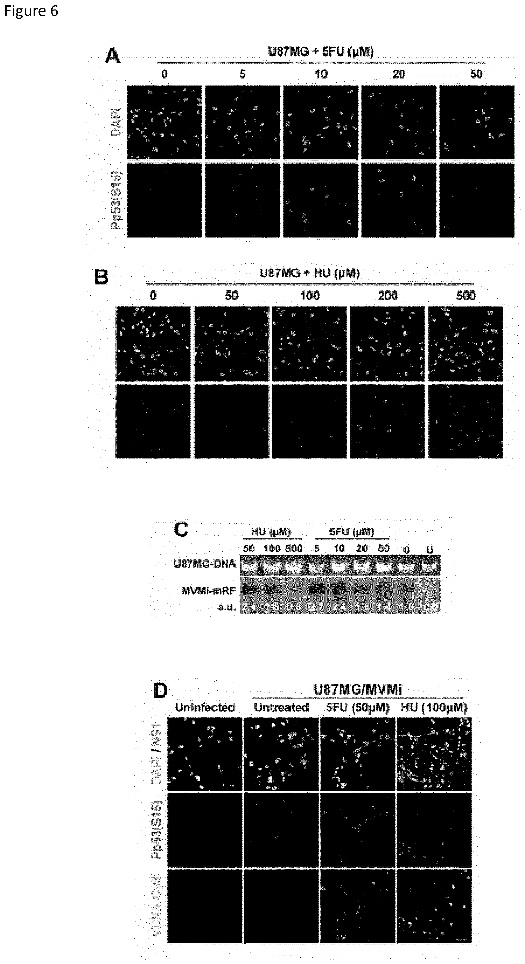

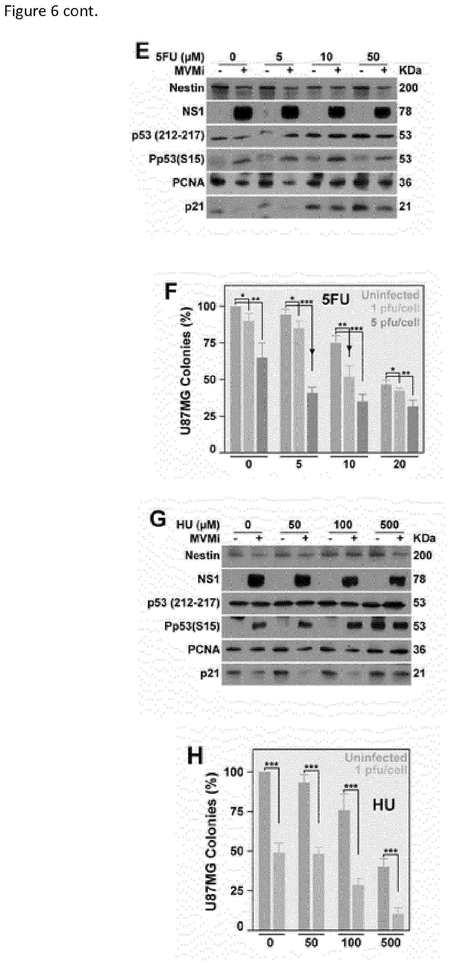

**Figure 6****.** CD effects on the infection of U87 MG glioblastoma cells with the parvovirus MVMi. **A, B.** IF-confocal analysis of the dose effect of 5FU and hydroxyurea (HU) on the p53-S15 levels in uninfected U87 cells. **C.** Dose effect of these CD on the levels of the main replicative intermediate (mRF) of the viral genome analyzed by Southern-blot. **D.** IF-confocal analysis of the effect of these CD on the levels of NS1, p53-S15 and virus genome replication. Dose effect of 5FU on the levels of NS1 and p53 signaling proteins measured in blot (**E**), and on cell viability determined by colony formation **(F).** Dose effect of HU on the levels of NS1 and p53 signaling proteins measured by blot (**G**), and on cell viability determined by colony formation (**H**).

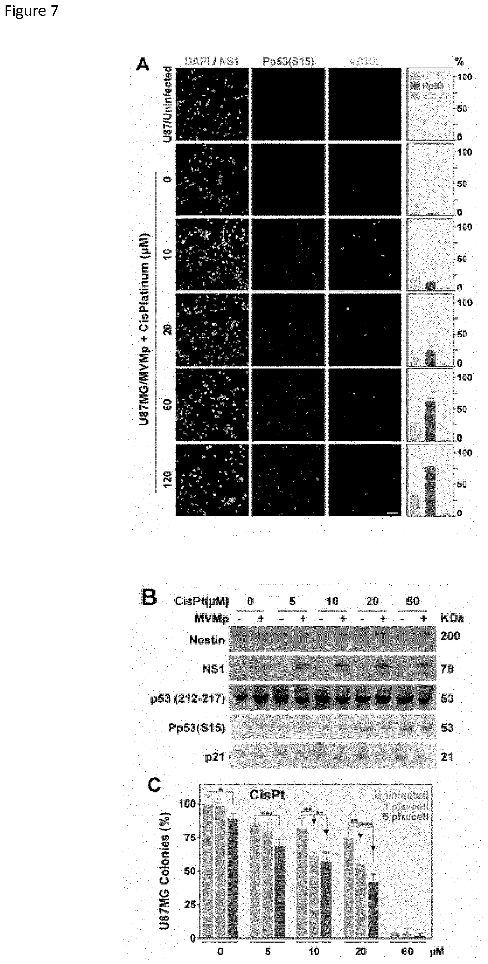

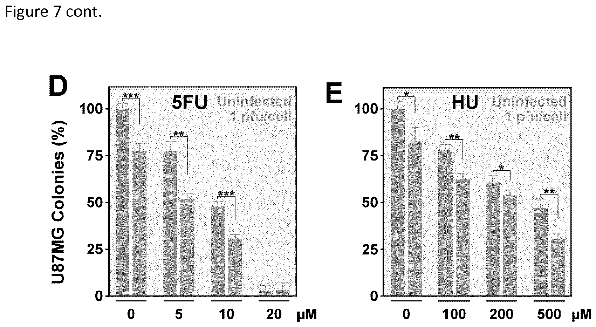

**Figure 7****.** CDs effects on the infection of U87MG cells with parvovirus MVMp. A. IF-confocal analysis of Cisplatin dose effect on the levels of NS1, p53-S15 and parvovirus genome replication. **B.** Western blot analysis of equivalent samples by measuring NS1 levels and several p53 signaling cellular proteins. **C, D** and **E.** Cell survival (measured by colony formation) in infections at different multiplicities (PFU/cell) with MVMp combined with the indicated doses of diverse CDs.

### Detailed description of the invention

### EXAMPLE 1. MATERIALS AND METHODS

### Example 1.1. Cells and cell cultures.

The human glioblastoma stem cells were obtained from tumor explants provided by the Neurosurgery service of the Ramón y Cajal Hospital in Madrid. Explants were diagnosed as glioblastoma grade IV by histochemistry performed by the Pathology service of the same hospital. In all cases, the informed consent of the patients was obtained and the approval of the Institutional Ethics Committee of the Ramón y Cajal Hospital in Madrid. Further research in tissue culture was authorized by the respective Ethics Committee of the Universidad de Madrid, and Centro de Biología Molecular Severo Ochoa (CSIC-UAM). The biopsies, collected at the foot of the operating room, were mechanically and enzymatically disintegrated, and finally the cell suspension was filtered to be cultivated in the DMEM medium: F12 (1: 1) supplemented with various factors.

On the other hand, the established cell lines of mouse, human cancers and other mammals were cultured in Dulbecco's Modified Eagle medium (DMEM) buffered with 0.3% NaHCO₃ and in atmosphere of 5% CO₂. The medium was supplemented with antibiotics (75 U/ml streptomycin, 75 µg/ml penicillin G), 2 mM L-Glutamine and 5% of fetal bovine serum (FCS) des-complemented at 56 ° C for 30 minutes. The following cell lines have been used:
- Human Glioblastoma:
   - U87-MG: glioblastoma-human astrocytoma (ATCC: HTB 14).
   - U373-MG: glioblastoma-human astrocytoma (RRID: CVCL_2219)
   - U251-MG: glioblastoma-human astrocytoma (RRID:CVCL_0021) (ECACC09063001).
- Other human cancers:
   - Hela, human cervical carcinoma (ATCC-CCl-2)
   - NB324K: human kidney fibroblasts of newborn transformed with the large T antigen 1 *Polyomavirus* SV40 (RRID: CVCLU409).
- Carcinogenic cell lines of other mammals:
   - COS-1, primate liver fibroblasts transformed by the *Polyomavirus* SV40 (ATCC CRL-1650)
   - A9: line derived from mouse L fibroblasts selected by the HGPRT phenotype. It shows a certain carcinogenic capacity when injected into mice (ATCC CCL-1.4).

### Example 1.2. Flow cytometry.

In all the experiments, a minimum of 30,000 events per sample were acquired in a FACSCantoII cytometer (BD Biosciences), which were pre-selected by a region based on their size and complexity (FSC and SSC parameters, respectively) in order to exclude cellular debris and other contaminating particles. The CellQuest software (BD Biosciences) was used to acquire the events and the FlowJo software (Tree Star) was used to analyze the data. The cell cycle analysis was in accordance to [Gil-Ranedo, J., Hernando, E., Riolobos, L., Dominguez, C., Kann, M., and José M. Almendral. 2015.The mammalian cell cycle regulates nuclear parvovirus capsid assembly. PlosPathogens, 11; 11 (6): e1004920]*.* For staining, cells were permeabilized with PBS + 0.1% triton X-100 for 10 minutes at room temperature, then blocked with the same buffer supplemented with 1% FCS for 20 minutes. Cells were re-suspended in PBS (pH 7.2) +0.5% BSA and the primary antibodies indicated in the figures were added followed by 1h stirring at 37 °C. Cells were washed in PBS and the secondary antibodies were added and incubated similarly. Two further washes in PBS were made before analyzing the samples in the flow cytometer.

In the purification of cells by flow cytometry (FACS) for the genetic analysis of TP53 (as shown in the **Figure 3E**), all solutions were prepared with Diethyl Pyrocarbonate (DPC) using distilled water free of nucleases (Gifco, distilled water DNase/RNase free 10977-035). Cells were permeabilized and incubated with antibodies as indicated above, but PBS buffers were supplemented with vanadate (RNase inhibitor), gelatin instead of BSA (allowing autoclave), and filtered. Before sorting, the cytometry equipment (Aria, BD) was extensively washed with autoclaved PBS-DEP pre-cooled at 4 °C, the circumvented cells were collected on sterile tubes (F15, Falcon) on ice, and Trizol was immediately added to inhibit degradation and next proceed with RNA purification.

### Example 1.3. Clonogenic analysis of cell viability.

The clonogenic capacity and cell viability were estimated by a colony formation assay based on [Rubio, MP, Guerra, S., and Almendral, JM 2001. Genome replication and postencapsidation functions mapping to the nonstructural gene restrict the host range of a murine parvovirus in human cells. J Virol 75 (23): 11573-11582].

### Example 1.4. Indirect immunofluorescence (IF).

Immunofluorescence analyses were performed on neurospheres of hGSCs (human glioblastoma stem cells) and established adherent cell lines as follows:
- Neurosphere staining. The cell spheres were fixed for 30 minutes with paraformaldehyde (PFA) 4%, then permeabilized for 10 minutes at room temperature with 0.1% Triton X-100 (Merck) in PBS, and blocked by incubating 20 minutes with 0.1% Triton X-100 and 10% fetal bovine serum (Sigma) in PBS. Neurospheres were incubated in the same buffer with the primary antibodies indicated in the figures for 1 h at 37 °C, and finished by four five-minute PBS washes. Then neurospheres were incubated with the secondary antibodies at appropriate concentrations in the same buffer and conditions, and finally stained with DAPI for half an hour and mounted embedding at 37 °C in 0.8% agarose-LGT prepared in PBS. Embedded neurospheres were applied as 200 microliters drops on pre-cooled large (2x 3 cm) coverslips and stored at 4 °C in the dark until confocal analysis.
- Adherent cells. The cell lines were seeded on coverslips, treated as indicated in the figures and fixed for 10 minutes with 4% paraformaldehyde. Staining with antibodies was carried out essentially as described above for the neurospheres. Cells were mounted with Fluoromount G (Southern Biotech). Preparations were kept at least 16 h at room temperature in the dark before observation under a microscope.
- Analysis of virus replication by FISH. This method was used to determine the degree of replication of the parvovirus MVM genome. The probe used was a mixture of three DNA oligonucleotides labeled at the 3' terminal end with the Cy5 fluorophore, and complementary to different sequences of the MVM genome. Cells adhered and fixed in 4% paraformaldehyde were permeabilized for 10 minutes with 0.2% Triton X-100 (Merck) in PBS and equilibrated for 5 minutes at room temperature in 2x SSC (0.3 M NaCl; 30 mM Na₂C₆H₅O₇, pH 7) supplemented with 15% formamide. Hybridization was with 500 ng/ml of each oligonucleotide in FISH buffer (5xSSC, 0.5% SDS, 10% dextran sulfate, 50% formamide) for 2.5 hours at 37 °C. The preparations were washed twice with 2xSSC, 15% formamide for 30 minutes. Finally, samples were blocked in 0.1% Triton X-100 and 1% FCS in PBS for 20 minutes at room temperature, and the immunofluorescence protocol described above was continued.
- Image captures. In neurosphere preparations, a confocal laser scanning microscope LSM510 META coupled to an inverted Axiovert200 microscope (Zeiss), or a multiphoton confocal laser scanning microscope LSM710 coupled to an inverted microscope AxioObserver (Zeiss) were used. For the analysis of preparations with dispersed cells, an Axiovert200 (Zeiss) inverted microscope coupled to a monochrome and color ccd camera was used. Whenever indicated, the multiphoton confocal laser scanning microscope LSM710 was used. The images were processed with Adobe Photoshop CS5 programs (Adobe Systems Incorporated) and ImageJ/FIJI (http://rsb.info.nih.gov/ij/) taking the same samples as a zero-signal reference without antibodies (auto-fluorescence control), or only with secondary antibodies.

### Example 1.5. Parvovirus MVM.

MVM belongs to the family *Parvoviridae,* genus *Protoparvovirus.* The prototypic strain of this virus (MVMp) was originally isolated from fibroblasts [Crawford, L. (1966) A minute virus of mice, Virology, 29, p. 605-612] and the so-called immunosuppressive strain (MVMi) from mouse lymphocytes [Bonnard, GD, Manders, EK, Campbell, DA, Herberman, RB and Collins, MJ (1976) Immunosuppressive activity of a subline of the mouse EL-4 Lymphoma, J Exp Med, 143 (1), pp. 187-205. DOI: 10.1084 / jem.143.1.187]*.* Only the MVMi strain is pathogenic in mice.

### Example 1.6. Production of Parvovirus MVM in culture.

The preparations of the parvovirus MVM (p, i) were obtained in NB324K cells following established protocols [Segovia, JC, Gallego, JM, Bueren, JA and Almendral, JM (1999) Severe leukopenia and dysregulated erythropoiesis in SCID mice persistently infected with the parvovirus minute virus of mice., Journal of Virology, 73 (3), pp. 1774-84], [Sánchez-Martínez, C., Grueso, E., Carroll, M., Rommelaere, J. and Almendral, JM (2012) Essential role of the unordered VP2 n-terminal domain of the parvovirus MVM capsid in nuclear assembly and endosomal enlargement of the virion fivefold channel for cell entry. Virology, 432 (1), pp. 45-56], [Gil-Ranedo, J., Hernando, E., Valle, N., Riolobos, L., Maroto, B. and Almendral, JM (2018) Differential phosphorylation and n-terminal configuration of capsid subunits in parvovirus assembly and viral trafficking, Virology, 518, pp. 184-194]. For this, we used plasmids containing the complete genome of MVMp and MVMi strains, including the palindromic sequences of the [Gardiner, MS and Tattersall, P. 1988. Mapping of the fibrotropic and lymphotropic host range determinants of the parvovirus minute virus of mice. J Virol 62 (8): 2605-2613] [Hirt, B., Colomar, MC and Beard, P. (1998) Two segments in the genome of the immunosuppressive minute virus of mice determine the host-cell specificity, viral control DNA replication and affect viral RNA metabolism., Journal of General Virology , 79, 581-586] which allows obtaining viral particles by transfection of cells [Sánchez-Martínez, C., Grueso, E., Carroll, M., Rommelaere, J. and Almendral, JM (2012) Essential role of the unordered VP2 n-terminal domain of the parvovirus MVM capsid in nuclear assembly and endosomal enlargement of the virion fivefold channel for cell entry. Virology, 432 (1), pp. 45-56]. Using virus directly produced by transfection (48h post-transfection), NB324K cells grown to confluence in ten P100 plates (a total of 5x10⁷ cells) were infected at a multiplicity of infection (MOI) of 0.005 plaque-forming units/cell (PFU / cell) in 1 ml of complete PBS (PBSc; PBS) with 0.9 mM CaCl₂ and 0.5 mM MgCl₂) with 0.1% FCS. After 1 h at 37 °C, the inoculum was removed and incubated in DMEM with 5% FCS for 5 h to allow internalization of the virus. Subsequently the adhered cells were detached with trypsin-EDTA, diluted in 400 ml of DMEM with 5% FCS and seeded onto fifty P100 plates. Cells were incubated until the appearance of cytopathic effect (approximately 5 days).

### Example 1.7. Purification of parvovirus MVM.

We followed protocols described by [Santarén, JF, Ramírez, JC and Almendral, JM (1993) Protein species of the parvovirus minute virus of MVMp strain: involvement of phosphorylated VP-2 subtypes in viral morphogenesis., Journal of Virology, 67 (9), pp. 5126-38], [Hernando, E., Llamas-Saiz, AL, Foces-Foces, C., McKenna, R., Portman, I., Agbandje-McKenna, M. and Almendral, JM (2000) Biochemical and Physical Characterization of Parvovirus Minute Virus of Mice Virus-like Particles, Virology, 267 (2), pp. 299-309]*,* [Sánchez-Martínez, C., Grueso, E., Carroll, M., Rommelaere, J. and Almendral, JM (2012) Essential role of the unordered VP2 n-terminal domain of the parvovirus MVM capsid in nuclear assembly and endosomal enlargement of the virion fivefold channel for cell entry. Virology, 432 (1), pp. 45-56] and [Gil-Ranedo, J., Hernando, E., Riolobos, L., Dominguez, C., Kann, M., and José M. Almendral. 2015. The mammalian cell cycle regulates nuclear parvovirus capsid assembly. PlosPathogens, 11; 11 (6): e1004920]. In short, the virus present in the medium was recovered by precipitation with 3.4% polyethylene glycol 6000 and 0.5 M NaCl overnight at 4 °C and subsequently centrifuged at 5000 rpm for 30 minutes in an angular Sorvall GSA rotor. To recover the intracellular virus, the cell pellet was resuspended in 50 mM Tris-HCl pH 7.5, 1 mM EDTA (TE) and subjected to three consecutive freeze/thaw cycles, after which 0.2% SDS was added and clarified at 8000 rpm, 10 min at 4 °C in a Sorvall HB4 swinging rotor. The virus recovered from the medium and the intracellular virus were pooled and centrifuged through a 20% sucrose cushion (Merck) in 50 mM Tris-HCl pH 8.0, 1 mM EDTA, 0.1 M NaCl and 0.2% SDS for 18h at 16000 rpm in a TST 28.38 rotor. The pellet was resuspended in TE with 0.2% Sarkosyl (Sigma) and banded to equilibrium in a CsCl gradient (ni = 1.371) run for 24 h at 50000 rpm and 15 °C in a TFT 80.13 rotor. Fractions of 0.5 ml were collected by syringe from the top of the gradients and the presence of empty capsids and DNA-filled viruses was determined by hemagglutination with mouse erythrocytes. Finally, the fractions containing the viruses were pooled and dialyzed against PBS. Purified virus was stored in aliquots at -70 °C.

### Example 1.8. Hemagglutination.

This method was used to estimate the amount of virus particles and follow up purifications. It was based on [Sánchez-Martínez, C., Grueso, E., Carroll, M., Rommelaere, J. and Almendral, JM (2012) Essential role of the unordered VP2 n-terminal domain of the parvovirus MVM capsid in nuclear assembly and endosomal enlargement of the virion fivefold channel for cell entry. Virology, 432 (1), pp. 45-56] and [Hernando, E., Llamas-Saiz, AL, Foces-Foces, C., McKenna, R., Portman, I., Agbandje-McKenna, M. and Almendral, JM (2000) Biochemical and Physical Characterization of Parvovirus Minute Virus of Mice Virus-like Particles, Virology, 267 (2), pp. 299-309]*.* For the hemagglutination (HA) assay, adult mouse blood was washed three times with phosphate saline (PBS), collecting the erythrocytes by centrifugation in a tabletop centrifuge at 1500 rpm for 5 min. After several washes the erythrocyte sediment was resuspended as 50% (v/v) in PBS and kept at 4 °C until use. The HA was carried out in U-profile microtest plates (Nunc). The samples to be evaluated were applied in a final volume of 100 µl in PBS and serial dilutions 1: 2 in PBS were made. Finally, 50 µl of 2 % erythrocytes in PBS was added to each well, the plate was gently shaken and kept at 4 °C in darkness for at least two hours. The title was obtained from the inverse of the highest dilution that maintains the hemagglutinating capacity.

### Example 1.9. Parvovirus MVM titration by plaques formation.

Based on [Gil-Ranedo, J., Hernando, E., Valle, N., Riolobos, L., Maroto, B. and Almendral, JM (2018) Differential phosphorylation and n-terminal configuration of capsid subunits in parvovirus assembly and viral trafficking, Virology, 518, pp. 184-194]. NB324K cells seeded 24h before in P60 plates at a density of 2.2x10e5 cells/plate were used. The culture medium was removed, cells washed in PBS with Ca++ and Mg++ (complete or PBSc), and the viral inoculum was added in 400 µl per P60 diluted in PBSc supplemented with 0.1% FCS. After one-hour adsorption at 37 °C with gentle agitation, the inoculum was removed and 7 ml of plating medium (DMEM, 10% FCS, and 0.6% agarose LM-GQT Pronadisa) equilibrated at 37 °C was added. After a 6-days incubation, the plates were fixed in 10% formaldehyde (Panreac) in PBS and stained with 0.2 % violet crystal (Panreac) in 10% formaldehyde prepared in PBS. The count of the number of lysis plaques multiplied by the dilution allows to obtain the infectious titer of the virus in plaque forming units per milliliter (PFU/ml).

### Example 1.10. Obtaining viral DNA.

The low molecular weight DNA from cells electroporated with plasmids, or infected with MVM, was obtained by a modified Hirt's method [Segovia, JC, Gallego, JM, Bueren, JA and Almendral, JM (1999) Severe leukopenia and dysregulated erythropoiesis in SCID mice persistently infected with the parvovirus minute virus of mice., Journal of Virology, 73 (3), pp. 1774-1784]. Briefly, the transfected cells were lysed in Hirt's solution (50 mMTris pH 7.5, 0.5% SDS, 10 mM EDTA) supplemented with 20 µg/ml tRNA *carrier* to ensure recovery, and digested with proteinase K (100 µg /ml) (Merck) for 2 hours at 37 °C. The reaction was adjusted to 1M NaCl and the genomic DNA was precipitated overnight at 4 °C. The enriched fraction of low molecular weight viral DNA was obtained from the supernatant after centrifuging the samples at 4 °C and 14 K rpm for 30 minutes in a microfuge (Eppendorff). This DNA was precipitated with 0.3 M NaCl and 2.5 volumes of absolute ethanol at -20 °C, washed with 70 % ethanol to remove salts, and resuspended in water or in 50 mM Tris pH 7.5 and 1 mM EDTA.

### Example 1.11. DNA electrophoresis and transfer to membranes.

Samples were electrophoresed 2-3 h at 60V on a 0.8% agarose gel (Gibco) in Tris-Borate-EDTA buffer (45mM Tris-borate, 1mM EDTA) with 5 µg/ml ethidium bromide (Boehringer) together with molecular weight markers (HindIII digest of phage Ø29 DNA) and controls of replicative forms and ssDNA of MVM. The transfer was made to a Nylon membrane (Hybond-N+, Amersham, Pharmacia) in 0.4 M NaOH overnight. Finally, the membrane was washed in a 2XSSC solution for 10 minutes at room temperature (20X SSC is: 3 M NaCl, 0.3 M sodium citrate) and dried 2 hours at 78 °C.

### Example 1.12. Hybridization with specific MVM probe.

The membrane was incubated in pre-hybridization solution (5X SSC, 5X Denhardts' solution [Ficoll (Ty400), Polyvinylpyrrolidone, BSA], 10 mM Tris-HCl pH 7.5, 0.5% SDS, 50% Formamide), to eliminate possible nonspecific binding, for four hours at 42 °C. Next, the solution was replaced by the hybridization solution, which is formed by the same components of the pre-hybridization solution together with the denatured probe. The probe was the full-length the MVM genome labeled *in vitro* to high specific activity with ³²P by "random priming" generally using dCTP-alpha ³²P and purified by a Sephadex G-50 spin-column. Hybridization was allowed at 42 °C for one or two days, and finally membranes were washed with a solution of 0.1X SSC and 0.5% SDS at 50 °C for three hours, before exposure to X-ray films.

### Example 1.13. Antibodies

The primary and secondary antibodies used in immunological techniques were:

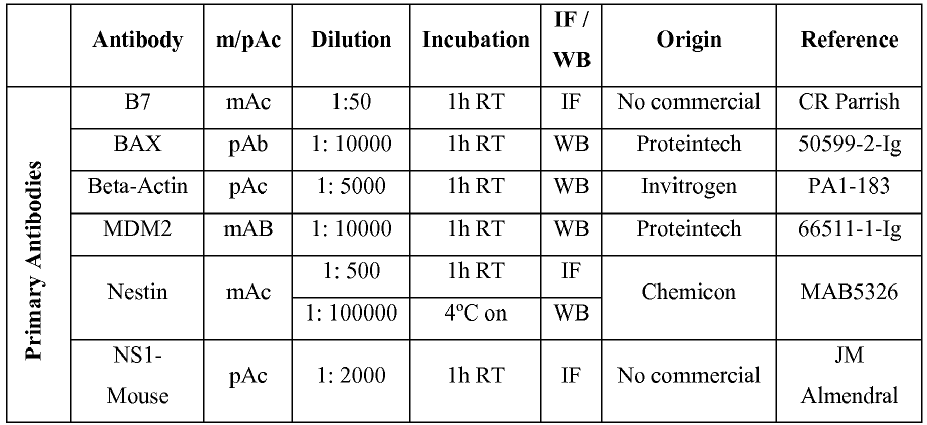

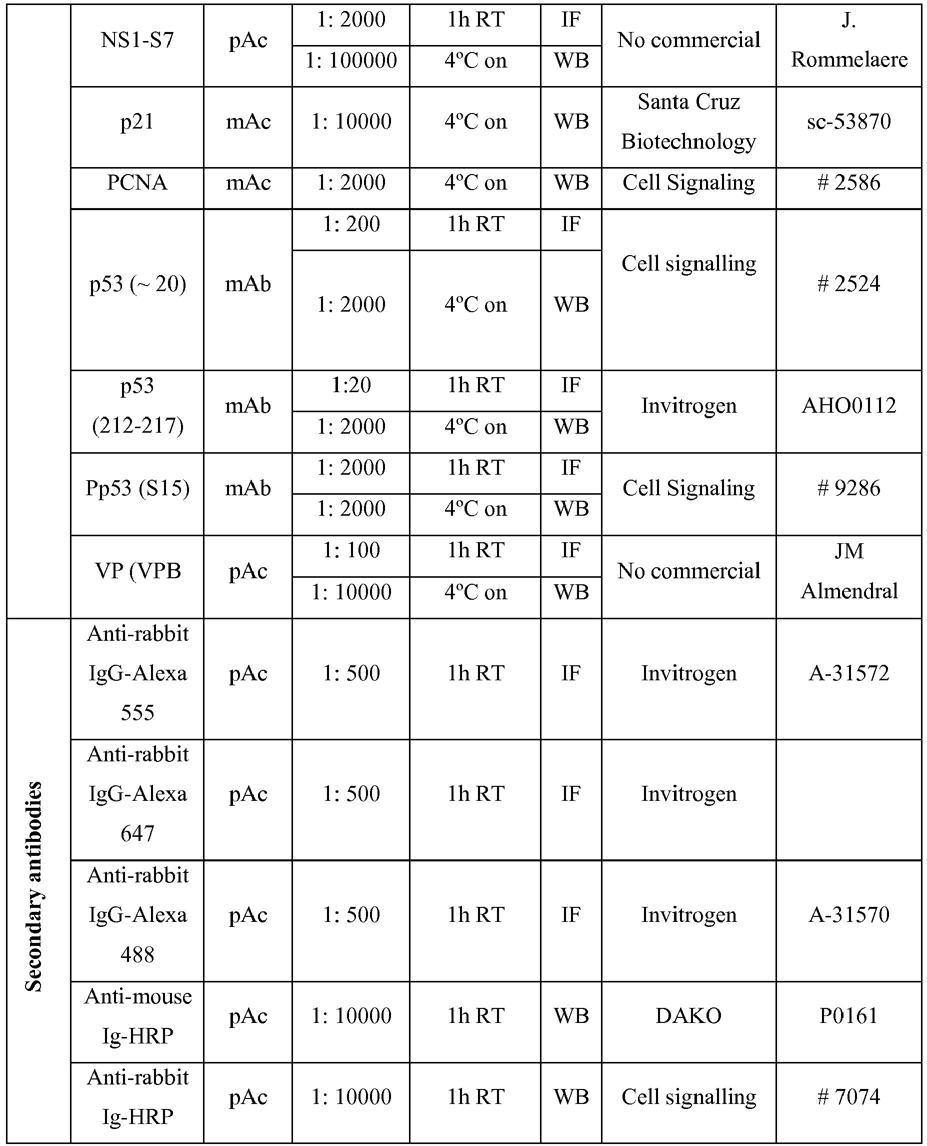

### Example 1.14. Chemical reagents.

Hydroxyurea was obtained from Calbiochem (Hydroxyurea cat 400046-5gm). 5-Fluoruracil (5FU) was obtained from Sigma (Ref F-6627-1G). Cisplatin from EMC Millipore (232120-50mg).

**Example 1.15. Protein analyses: SDS-PAGE and transfer to membranes.** Protein samples, once denatured by boiling for five minutes in loading buffer (10% glycerol (Merck), 5% β-mercaptoethanol (Merck), 0.002% bromophenol blue (Merck), 0.5M Tris-HCl pH 6.3, 0.4 % SDS), were resolved by denaturing gel electrophoresis of 8% polyacrylamide. Electrophoresis was carried out in a Tris-Glycine buffer (25 mM Tri-HCl (Serva), 192 mM Glycine (Gibco), 0.1% SDS) for two-four hours at 100 V in minigels (10x10x0.1 cm), with molecular mass markers run in parallel ("Prestained SDS-PAGE Standards, Broad Range" (Biorad), or "Protein Molecular Weight Standards, Broad Range", Amersham). The samples were transferred to nitrocellulose memebrane (Schleicher and Schuell) in transfer buffer (25 mM Tris base, 192 mM glycine, 0.1% SDS, 20% methanol) for one hour at 100 V (Trans-blot electrophoretic transfer Cell, Biorad).

### Example 1.16. Detection of proteins blotted to membrane ("western blot").

The membrane was hydrated in TBS-T buffer (20 mM Tris pH 7.5, 140 mM NaCl, 0.1% Tween 20) and incubated under shaking for one hour at 4 °C in TBS-T with 10% fetal bovine serum (FBS). After washing with TBS-T it was incubated with the primary antibody diluted in TBS-T with 1% FBS and 1% NP40, for 24h at 4 °C. After thorough washing, the secondary antibody was added at incubated for 1h at RT. Finally, the membrane was washed with TBS-T and TBS (without Tween 20), revealed with the ECL system ("Enhanced Chemiluminiscence", Amersham) and exposed to autoradiograpy (Kodak).

### Example 1.17. Plasmids.

To express the p53 protein in cell cultures the pCMV- neo-p53 plasmid was used [ Baker SJ, Markowitz S, Fearon ER , Willson JK, a nd Vogelstein B. Suppression of human colorectal carcinoma cell growth by wild-type p53. Science 1990, 249 (4971): 912-5], kindly provided by J. Paramio (Ciemat, Madrid). The plasmid named "Helper" and the plasmid to express the E4orf6 oncogene of Adenovirus [Winter, K., von Kietzell, K., Heilbronn, R., Pozzuto, T., Fechner, H., and S. Weger. (2012). Roles of E4orf6 and VA I RNA inadenovirus-mediesated stimulation of human parvovirus B19 DNA replication and structural gene expression. J. Virol. 86, 5099-5109] were kindly donated by S. Weger (Charité, Berlin). Plasmids were transfected into cells by a chemical (Jetpaid) or physical (electroporation) method depending on cell type.

### Example 1.18. Sequencing of the TP53 gene.

Cell samples were processed to obtain total RNA using Trizol and conventional protocols. The RNA was then copied to cDNA using Reverse Transcriptase and random primers. The thus obtained cDNA was used to sequence the human or mouse TP53 gene using the TP53 sequence and primers listed in the Annex. Methods followed were the conventional Sanger sequencing, or massive sequencing (NGS) for the RNA samples obtained from FACS-sorted GSs (see Figures 2 and 3). Both methods of sequencing were performed by the Parque Científico de Madrid (PCM) using the equipment and protocols available in this facility.

### EXAMPLE 2. RESULTS.

### Example 2.1. The expression of the parvovirus MVM major NS1 cytotoxic protein in human glioblastoma stem cells (GS) correlates with p53 induction.

**Figure 1** (left) shows the p53 response in GS from three patients (# 5, 7 and 8) infected by the MVMp or MVMi strains. In uninfected neurospheres, p53 staining is weak and homogeneous. After infection the expression of NS1, the major nonstructural cytotoxic virus protein, was detected in a variable % of GS depending on the patient, but in all three cases p53 is induced strongly and specifically in the NS1+ cells. When infections of GS from four patients were analyzed in western blot (**Figure 1****,** right), the p53 induction of p53 is not evident by the signal background coming from most cells not expressing NS1. Only in GS7, more permissive to MVM, this induction is patent. Therefore, GS cells induce a genuine DNA damage response (DDR) involving p53, in response to MVM infection.

### Example 2.2. Those GS permissive to the parvovirus MVM genome replication induce p53 modified by phosphorylation at the Serine 15 residue.

We next studied whether the DDR mounted by the GS in response to the parvovirus MVM involves, in a cell-type and/or virus-strain dependent, the modification at the Serine 15 residue of p53 by phosphorylation (Pp53-S15). In uninfected GS neurospheres, a basal expression of Pp53-S15 was observed, with some prominent cells in GS5 and GS7, but when infected by MVMp the Pp53-S15 is clearly induced in a significant fraction of the cells expressing NS1 (**Figure 2A**). An estimate of the basal expression percentages, and of the Pp53-S15 induction in response to MVM strains infection -in GS as well as in various established lines- are shown in **Figure 3B****.** However, the virus genome replication in the neurospheres occurs preferentially in those Pp53-S15+ GS cells **(****Figure 2A****,** *below).* This correlation NS1+/Pp53-S15+ was quantitatively confirmed by flow cytometry (**Figure 2B**), since in the NS1+ populations of GS from two patients infected by MVMp, the synthesis of viral DNA (vDNA+) is preferably carried out in cells expressing high levels of Pp53-S15.

### Example 2.3. Transformed cell lines of distinct origins, including various types of human cancers, which are permissive to NS1 expression and sometimes to MVM genome replication, harbor p53 mutated and/or altered phenotypically, usually by phosphorylation at Ser15.

To extend the previous study to other types of cancers, as well as to the cells used to grow these viruses, several human and other mammals cell lines were infected with the two virus strains (MVMp, MVMi) and analyzed for the NS1 expression and virus genome replications, in relation to the presence of mutations in the TP53 gene and to post-translational modifications of the p53 protein. The results obtained in these analyses were:
- The A9 cell line of immortal mouse fibroblasts, commonly used to grow MVMp virus [Gardiner, EM and Tattersall, P. 1988. Mapping of the fibrotropic and lymphotropic host range determinants of the parvovirus minute virus of mice. J Virol 62 (8): 2605-2613] [Gil-Ranedo, J., Hernando, E., Riolobos, L., Dominguez, C., Kann, M., and José M. Almendral. 2015. The mammalian cell cycle regulates nuclear parvovirus capsid assembly. PlosPathogens, 11; 11 (6): e1004920] infected with MVMp and synchronized with thymidine, were analyzed at 9 h post-release of this arrest, when the synthesis of macromolecules of the virus reaches its maximum values [Gil-Ranedo, J., Hernando, E., Riolobos, L., Dominguez, C., Kann, M., and José M. Almond tree 2015.The mammalian cell cycle regulates nuclear parvovirus capsid assembly. PlosPathogens, 11; 11 (6): e1004920]*.* It was observed that while the cell cycle of uninfected A9 cells accumulated under these conditions at G2 (**Figure 2C****,** upper), a significant fraction of the infected cells was retained before G2. Although close to 40% of the cell population did not express NS1, all cells expressing NS1 had progressed to G2. More importantly, only half of those NS1 expressing cells are permissive to the synthesis of the virus DNA (vDNA). P53 was induced in response to infection, and level of synthesis corresponded to that of NS1 (**Figure 2C****,** below). In these cells the Pp53-S15 constitutive levels were undetectable (see also **Figure 3C**), but were strongly induced in response to infection, and those vDNA+ cells have higher p53-S15 induction levels **(****Figure 2C**, middle panel).
   To analyze the marked difference between the NS1+ cells allowing or not vDNA synthesis, we proceeded to sort these two cell populations and to characterize their *TP53* genetic. Sorted A9 NS1+ cells behaved again distributed in two populations vDNA+/- of similar size (**Figure 3E****,** left). Remarkably, the TP53-G665 C mutation (which determines the amino acid change V170 L) was detected in the expressed mRNA in the two A9 populations, as well as in the initial unsorted A9 population, but not in control non-transformed mouse fibroblasts non-permissive to MVM infection (**Figure 3H**). Therefore, A9 cells have the nonsense V170L mutation (corresponding to V173L in the human TP53 gene), which may be required for the expression of NS1 (see more below). The MVMp genome replication is mainly confined to a A9 cell subpopulation in which infection induces p53 phosphorylated at Ser15.
- Human and Primate cell lines transformed by oncogenic viruses (**Figure 3A****,** upper), expressing oncogenes that inactivate or functionally alter p53. These were three: NB324K, human newborn kidney cells transformed by the oncogenic polyomavirus SV40 T antigen [Shein, HM, Enders, JF and Levinthal, JD (1962). Transformation induced by simian virus 40 in human renal cell cultures. II. Cell-virus relationships. Proceedings of the National Academy of Sciences of the United States of America, 48, pp. 1350-7]*.* The sequence of the TP53 gene in these cells determined from cDNA had no mutations (data not shown), but they did show Pp53-S15 constitutive alteration, p53 induction correlating with NS1 expression, and virus genome replication at high levels in most cells (**Figure 3A** and **Figure 3D**), although the Pp53-S15 expressing levels in virus replicating cells varied. The results were similar in COS-1 cells (primate fibroblasts also transformed by SV40), and Hela (obtained from human cervical cancer induced by Papillomavirus infection), although the virus genome replication and Pp53-S15 constitutive levels were generally lower (**Figure 3A**).
- Cell lines established from glioblastoma patients.
   - U373-MG and U251-MG. These cells harbor the TP53-g849a mutation that encodes the R273H amino acid change in p53, a mutation well documented in the literature that we confirmed by Sanger sequencing of their mRNA (data not shown). These cells are poorly permissive to the two MVM (p, i) strains, as only a small fraction of the population induces NS1 at low levels, coinciding with a p53 rise (**Figure 3A** and **Figure 3D**). These glioblastoma cell lines show constitutive Pp53-S15 alteration, though at basal levels, and they sustain MVM (p, i) strains genome replication exclusively in those low number of Pp53 -S15+ cells (**Figure 3A**).
   - U87-MG. Genetic alterations in the *TP53* mRNA could not found in this cell line (data not shown), as described earlier in the literature, nor they showed the Pp53-S15 modification constitutively (see **Figures 4****,** **6** and **7****).** However, the U87-MG expressed p53 protein must have other type(s) of post-translational modification(s) since a commercial antibody (Invitrogen) raised against the 212-217 amino acids domain did recognize it (as well as other p53 species in other cell types), whereas other commercial antibody of different specificity (Cell Signaling) obtained against a peptide from the environment of residue 20, did not recognize p53 (**Figure 3F**). Notably U87-MG cells were mostly permissive to the expression of the NS1 protein of MVMi, but not that protein of MVMp, although MVMi did not replicate its genome nor induced Pp53-S15+ (**Figures 4** and **6**).

### Example 2.4. Expression of the cytotoxic NS1 protein of parvovirus MVM requires TP53 mutations or p53 protein modifications.

Although the correlation between genetic alterations in TP53 and/or modifications in the p53 protein with the expression of the NS1 protein of MVM was consistent in several transformed cell lines (**Figures 1-3**), this evidence did not demonstrate a causal relationship between both phenomena. To address this important issue, we proceeded to overexpress the p53 protein from the pCMV-neo-p53 plasmid *[*Baker SJ, Markowitz S, Fearon ER, Willson JK, Vogelstein B. Suppression of human colorectal carcinoma cell growth by wild-type p53. Science 1990, 249 (4971): 912-5*]* both in mouse A9 cells that possess the TP53-V173L mutation (actually the equivalent V170L mouse mutation) and NB324K cells that lack mutations in TP52 but show highly phosphorylated p53-S15 (**Figure 3A** and **C**). As shown in **Figure 3G****,** NS1 expression upon transfection with carrier DNA (prepared from salmon sperm) was very high in both cell types, while p53 levels were basal or undetectable. However, when p53 was over-expressed by the pCMV-neo-p53 transfection, an absolute inhibition of NS1 expression was caused. Those mouse A9 and human NB324K cells overexpressing transfected wtp53 above the characteristic constitutive levels, completely exclude the accumulation of the viral NS1 protein (see pTP53 panels in **Figure 3G**). Therefore, the expression of NS1 requires functional alterations of p53 induced by genetic mutations in TP53 or post-translational modifications in the p53 protein.

### Example 2.5. The parvovirus MVM expresses cytotoxic proteins and replicates its genome preferably in human glioblastoma stem cells (GS) harboring genetic alterations in TP53.

The analyses with established lines supported the association found between the Pp53-S15 modification and the permissiveness to MVM infection in GS cells (**Figure 2A**), and further suggested that this relationship could stand from a genetic basis. Therefore, to address this issue in depth, the genetic status of TP53 was analyzed in the GS of the four patients. For this, samples from control uninfected, MVMp or MVMi infected, as well as GS infected by MVMp and further sorted (FACS, see Material and Methods) for the NS1 and Pp53-S15 expression, were obtained. **Figure 3E** illustrates the restrictive windows (gates) chosen (to ensure purity) of the NS1 +/Pp53-S15+ and NS1+/Pp53-S15 cell populations submitted to genetic analysis. From all these samples polyA+ mRNA was purified, copied to cDNA and amplified by PCR across the TP53 gene. These amplicons were sequenced by new generation sequencing (NGS, **Figure 2D** and **E**) and confirmed by the conventional Sanger sequencing method (**Figure 3I**). The various mutations in TP53 that were detected are described below (discussed in the N to C direction of the p53 protein):
- The P72R Mutation. The c215g mutation that involves the amino acid residue P72R change (nonsense mutation) in the proline rich domain (PRD) of p53 (see **Figures 2F** and **3J**), is clearly apparent in the infected and FACS-sorted (+/+ and +/-) GS7 populations, as well as in a significant proportion of both equivalent GS8 sorted populations, and in a very low proportion of GS5 cells allowing NS1 expression (+/-). Therefore, the p53-P72R change benefits the MVMp transcription and replication, although to a different degree depending on the GS context GBM patient. It is interesting that this mutation has been described in a single sample of GBM by the Cosmic database (**Figure 2F**), but it is a common mutation in hematopoietic and lymphoid tumors, in particular P72R is by far the most frequent TP53 mutation in Acute Myeloid Leukemia (AML) (**Figure 3J**).
- The V173L mutation corresponds to the V170L change that we have identified in the A9 mouse fibroblast cell line. This mutation has been described in two GBM samples (**Figure 2F**), and it is localized in the "hotspot" region of the DBD domain of p53 where more mutations have been found (**Figure 3J**). The V173L change is implicated in diverse cancers but particularly at high frequency in rectal adenocarcinoma and lung adenocarcinoma (**Figure 3J**).
- The G245S mutation. It was found by NGS that the GS from patients 8 and 9 have the TP53 gene mutated in the position g733a, what entails the amino acid change G245S (nonsense mutation) in all cells and all samples (sorted and total populations regardless of MVM infection) of these patients (**Figure 2D****, E**). The mutation was confirmed in the same samples by Sanger sequencing (**Figure 3I**). The general presence of the mutation suggests that does not benefit the virus gene expression by itself. However, this TP53-g733a mutation also appears in a low proportion of the GS5 cells (**Figure 2E**), exclusively in those infected and sorted (+/+ and +/-), which point out that the p53-G245S change may contribute to MVM transcription and replication in these GS5 cells. It is important to note that this mutation has already been described at high frequency in the updated GBM genetic databases (**Figure 2F**), in consistency with its location within the "hot spot" region of p53, the DBD domain. Furthermore, the p53-G245S change is one of the most frequent mutations in the multiple types of human cancer analyzed for TP53 genetics (**Figure 3J**), as for example bladder cancer, rectal and lung adenocarcinomas (**Figure 3J**).
- The E258K mutation. The g772a mutation, which entails the E258K amino acid change (missense mutation), was observed by NGS in the low percentage of the infected and sorted GS7 population expressing NS1, strongly suggesting that it contributes to viral transcription in these GBM stem cells. This mutation was not previously described in the current GBM genetic databases (**Figure 2F**), although it is also localized in the "hotspot" p53 DBD domain, where more mutations have been found. Indeed the E258K mutation is common in many types of cancers (**Figure 2F****,** lower), and it stands out as a very common mutation in bladder carcinoma (**Figure 3J**).
- The R273H mutation. This nonsense mutation was not found in the primary GS of the four patients that we have analyzed (**Figures 2** and **3**), although it is very common in GBM genetic databases (**Figure 2F**). Noteworthy, the p53-R273H mutation is currently highlighted as the most frequent in the overall thousands of human cancers studied (see examples in **Figure 3J**). Although the failure to identify the R273H mutation in the four GS could be due to the limited number of samples, our study rather suggests that this mutation *per se* poorly benefits the MVM infection. This hypothesis is endorsed by the fact that the U373MG and U251MG glioblastoma cell lines harboring this mutation, are poorly permissive to MVMp as well as to MVMi infections (**Figure 3A****,** and **5**). However, MVMp makes very good synergy with chemotherapeutic drugs in the cytotoxic infection of the U373MG glioblastoma line (**Figure 5****,** see below), strongly suggesting that genotoxic chemotherapy treatments of GBM and other cancers bearing the p53-R273H mutation in combination with MVM may bring very important clinical benefits.
- The S366A mutation. The t1096g mutation, which entails the S366A amino acid change (a nonsense mutation), was observed by NGS in the global and sorted GS5 and GS7 populations independently of MVM infection. This suggests that this mutation by itself neither benefits nor inhibits parvovirus MVM infection. It is unlikely that this mutation contributes to some extent with others in the GS7 and GS5 permissiveness to MVM infection, although this possibility cannot be ruled out with the available data. The mutation S366A resides at the p53 carboxy-terminus, and it has not been described in GBM samples (**Figure 2F**). Indeed, its overall role in human cancer seems poorly relevant (**Figure 3J****,** below).

In summary, the following relevant conclusions can be drawn from this analysis:
- All GS populations expressing the NS1 cytotoxic protein, which may replicate the MVMp genome to different levels, harbor in a certain percentage some TP53 mutation, either individually or accompanied by other(s).
- The G245S and the R273H mutations favoring MVM infection in GS and glioblastoma cell lines respectively, are the most frequent p53 mutations found in GBM and other genetically characterized human cancers, which underscores the importance of our analysis.
- The P72R, V173 L, and E258K mutations, were previously undescribed or found at very low frequency in GBM. However, they do appear at high frequency in other cancer types, which may allow extending the MVM-based therapy other cancers.
- The P72R (except one case) and E258K mutations have not been previously described in GBM, yet they become detected in GS by NGS upon MVMp infection, which could allow the use of MVM for diagnosis of minor though clinically relevant p53 mutations in tumors.
- GS harboring TP53 mutations that correlate with NS1 expression also induce Pp53-S15 phosphorylation, which should promote virus transcription and replication.
- Therefore, among the large genetic heterogeneity of the GS populations, even being most cells wtTP53, the MVM specifically targets in the cytotoxic infection, and sometimes also in the virus genome replication, the low GS proportion harboring genetically altered *TP53.*
- The most frequent TP53 mutation in human cancer, R273H, does not make cells permissive to MVM by itself, but it does promote however an important synergistic cytotoxicity of the virus with chemotherapy (see more below).

### Example 2.6. The exogenous expression of oncogenes that alter p53 increases the permissiveness of GS and glioblastoma cell lines to MVM infection.

The high permissiveness to NS1 expression and MVM genome replication in cancer lines with constitutive Pp53-S15 staining and expressing viral oncogenes (**Figure 3A**, upper), prompted us to investigate a possible causal connection between both features, if wtTP53 cells could be made susceptible to MVM by exogenously altering functionally p53. **Figure 4A** shows that NIH3T3 mouse fibroblasts not permissive to MVMp infection substantially increase NS1 expression and virus genome replication upon transfection by a so called "helper" plasmid, which inactivates p53 by a degradation mediated by the expression of the E1A, E1B, and E4 orf6 adenovirus oncogenes, and inactivate PKR by the VA-RNA I [Winter, K., von Kietzell, K., Heilbronn, R., Pozzuto, T. Fechner, H., and S. Weger. (2012). Roles of E4orf6 and VA I RNA inadenovirus-mediesated stimulation of human parvovirus B19 DNA replication and structural gene expression. J. Virol. 86, 5099-5109]*.* In parallel (**Figure 4D**), MVMp infected GS5 cells, mounting a high DDR in response to NS1 expression, substantially increase the viral genome replication if they are transfected with the "helper" plasmid or another plasmid only expressing the E4orf6 oncogene [Winter, K., von Kietzell, K., Heilbronn, R., Pozzuto, T., Fechner, H., and S. Weger. (2012). Roles of E4orf6 and VA I RNA in adenovirus-mediated stimulation of human parvovirus B19 DNA replication and structural gene expression. J. Virol. 86, 5099-5109]*.* The results were confirmed in GS8 cells, which are very restrictive to MVMp replication although induce p53 in NS1 expressing cells (**Figure 4B**), but a significant increase in MVM replication is observed when oncogenes are expressed by transfection (**Figure 4C**). This experimental strategy was translated to the MVMi infection of U87-MG glioblastoma cells, which possess a wtTP53 sequence but express a post-translational modified p53 protein (**Figure 3**). Consistently, oncogenes expression also resulted in a significant increase in virus genome replication measured by IF-confocal (**Figure 4E**, left) and cytometry (**Figure 4E**, right). Therefore adenovirus oncogenes functionally inactivating p53 increase MVM replication in GSs and glioblastoma cell lines with wtTP53.

### Example 2.7. Chemotherapy drugs altering p53 phosphorylation increase MVMp gene expression, replication, and ability to kill U373-MG glioblastoma cells harboring the TP53-R273H mutation, very frequent in multiple types of human cancers.

As MVM infection correlated with Pp53-S15 expression (**Figure 2** and **3**), we studied whether the virus infection can benefit from a combination therapy with chemotherapeutic drugs (CD) of common use in clinical regimes (5FU, HU and Cis - Pt). For example, CisPt is a commonly used drug in clinical regimes against multiple human cancers (Dasari, S., and Tchounwou, PB. Cisplatin in cancer therapy: Molecular Mechanisms of action. Eur J Pharmacol. 2014 October 5; 0: 364-378). For this study, we used human glioblastoma cell lines with two genetic backgrounds. In the first study, U373MG carrying the R273H mutation frequently present in many types of human cancers [Muller, PA, and Vousden, KH 2013. P53 mutations in cancer. Nat. Cell Biol. 13, 2-8] [Brennan, CW et al. The somatic genomic landscape of glioblastoma. Cell 155, 462-477], moreover in a deep search in current databases performed by us it appears as the most frequent in GBM (**Figure 2F**) and cancer in general (**Figure 3J**). The **Figure 5A** shows by flow cytometric analysis of MVMp infected U373MG (that allows to quantitatively determine the levels of protein expression in cells), an increase in the percentage of cells expressing the NS1 protein, as well as in the level of expression, in combined treatments with a single dose of the CDs with respect to the simple infection. The CisPt treatment determines the highest increase in both parameters of NS1 expression. This benefit is most likely linked to the generalized Pp53-S15 induction in all cells caused by CisPt, which is also observed in treated and non-infected cells. This effect was confirmed by IF-confocal (**Figure 5B**).

Consequently, the CisPt dose effect on different MVMp life cycle parameters, and on p53 phosphorylation and functional signaling in U373-MG cells, was analyzed. For this, the U373-MG cells treated with Cis-Platinum doses (10-120 microM) for 1 hour at 37 °C were inoculated with MVMp to allow adsorption, and then of the infection was allowed to progress for 24 hours. Cells were sampled and processed for virus macromolecular markers and Pp53-S15 determinations. A significant increase in the Pp53-S15 levels was observed by IF-confocal across all the assayed 5-20 microM range of Cis-Pt doses (**Figure 5C**), in a coordinated manner to the increase (%) of the NS1 expression and virus genome (vDNA) replication with respect to the basal infection. Of note, the virus genome replication levels benefitted from 10 microM CisPt but not from higher doses, which seems to damage the vDNA synthesis machinery. The increasing NS1 levels across the 5 to 20 microM CisPt doses was confirmed by western blot (**Figure 5D**), which also reflected alterations in the levels of the p21 and MDM2 factors related to p53 functional signaling.

We finally analyzed whether these MVM/CisPt cooperative effects impact cancer cells viability, and therefore whether they imply a therapeutic significance. As seen in **Figure 5E****,** the simple 1-hour treatment with CisPt progressively decreased the viability (clonogenic assay) of U373-MG cells, in correspondence with its common use as chemotherapy drug in the oncology clinic. Moreover, MVMp singly inoculated at two MOI also decreased cell viability to some extent. However, when CisPt and MVMp were applied in combination, the cell viability decreased very significantly in an additive manner (**Figure 5E****,** 5 and 10 microM CisPt). This joint cooperation between the drug and the virus in killing human U373-MG glioblastoma cells, which harbor the TP53-R273H mutation, can lead to important therapeutic benefits in the treatments against the multiple human cancers carrying this mutation (see **Figure 3J**).

### Example 2. 8. The two MVMp and MVMi parvovirus strains, in combined therapy with CDs, increase their gene expression and replication, and their ability to kill U87-MG human glioblastoma cells harboring p53 post-translational modifications.

In a second study, this analysis of CD/MVM combined therapy was extended to the U87-MG human glioblastoma cell line, which lacks TP53 mutations but expressed post-translationally modified p53 protein forms (**Figure 3F**). In the first analysis, a possible CD/MVMi cooperation was studied. At basal state the U87-MG cells did not show significant Pp53-S15 signal, MVMi expressed high NS1 levels but failed to replicate its genome (**Figure 6D**). However, HU and 5FU treatments of uninfected U87-MG cells substantially increased *per se* p53 phosphorylation at the Ser15 residue (**Figure 6A****,B**). Consistent with this effect, when these CDs were administered at selected doses they proportionally increased the replication levels of the virus genome in these cells (**Figure 6C****, D**). As shown in **Figure 6F** and **H****,** this vDNA synthesis increase corresponded to a greater capacity of the MVMi to kill U87-MG cells in combination with 5FU (whose effects reached synergy levels) and with OH-U (additive effects). **Figure 6E** and **G** show as the increased Pp53-S15 phosphorylation that accompanies the infection (in respect to uninfected cells) is higher at the CD doses bringing best benefit for the virus, which also determine a more patent decrease in the p21 levels (a functional marker of p53).

In a second analysis the CD/MVMp cooperation was analyzed. MVMp infection of U87-MG (**Figure 7A**) yields a very low proportion of NS1+ cells even at high multiplicity (MOI 10). However, when cells were treated with different Cis-Pt doses prior infection, a very significant increase in the percentage of NS1+ cells was observed (**Figure 7A**). This increase was consistent with that of the Pp53-S15 levels correlating with the CisPt dosis used. The NS1/Pp53-S15 coordinated increase was also demonstrated by western-blot (**Figure 7B**), which also reflected p21 alterations. As described above for U373MG, the MVMp genome replication (vDNA) also benefitted from the 10 or 20 microM Cis-Pt treatment, but not from higher doses, suggesting an increased sensitiveness of the replicative over the transcriptional virus machineries to CisPt (**Figure 7A**). The increase in the % of NS1⁺ cells corresponded to the greater capacity of MVMp to kill U87-MG cells in combination with CisPt, which reached levels of synergy at the 10 and 20 microM doses (**Figure 7C**). Interestingly, the decrease in p21 levels (a functional marker of p53) in the infected cells was more patent at these doses (**Figure 7B**). Finally, combining MVMp with 5FU (**Figure 7D**) and HU (**Figure 7E**) also yielded consistent additive effects in U87-MG cells killing at the doses at which these CD induce higher Pp53-S15 levels in uninfected cells (see **Figure 6A** and **B** respectively).

## Claims

1. Viral particle comprising a nucleotide sequence consisting essentially of: SEQ ID NO: 1, SEQ ID NO: 2, SEQ ID NO: 3 or SEQ ID NO: 4, for use in the treatment of cancer, wherein the cancer is **characterized by** presenting mutations in the gene *TP53* and/or post-translational modifications in the p53 protein.

2. Viral particle for use, according to claim 1, wherein the post-translational modifications of the p53 protein are constitutive, induced by genotoxic chemotherapy drugs, or induced by oncogenic viruses.

3. Viral particle for use, according to any of the claims 1 or 2, wherein the post-translational modification of the p53 protein consists of the phosphorylation of the Ser15 residue.

4. Viral particle for use, according to any of the claims 1 to 3, wherein the mutation of the *TP53* gene is selected from the group comprising: R273H, P72R, E258K, G245S and/or V173L.

5. Viral particle for use, according to any of claims 1 to 4, wherein the mutation of the *TP53* gene is in the DBD or PRD regions the p53 protein.

6. Viral particle for use, according to any of claims 1 to 5, wherein the genotoxic chemotherapy drug that induces the post-translational modifications in the p53 protein is selected from the group comprising: cisplatin, hydroxyurea, 5-fluoruracyl, gemcitabine or cytosine arabinoside.

7. Viral particle for use, according to any of claims 1 to 6 , wherein the cancer is selected from the group comprising: glioma, glioblastoma, acute myeloid leukemia, lung adenocarcinoma, bladder carcinoma or rectal adenocarcinoma, which present the R273H, P72R, E258K, G245S and/or V173L mutations in the *TP53* gene .

8. Viral particle comprising a nucleotide sequence consisting essentially of: SEQ ID NO: 1, SEQ ID NO: 2, SEQ ID NO: 3 or SEQ ID NO: 4 for use, according to any of claims 1 to 7, in combination with one genotoxic chemotherapy drug, wherein the viral particle is administered simultaneously, after, or before the chemotherapy drug.

9. Viral particle for use, according to claim 8, in combination with a genotoxic chemotherapy drug in the treatment of a type of cancer **characterized by** presenting the R273H mutation in the *TP53* gene, selected from the group comprising: lung cancer, cancer esophagus, liver cancer, pancreatic cancer, bladder cancer, colorectal cancer, prostate cancer, glioblastoma, glioma, head and neck cancer, breast cancer, stomach cancer, ovarian cancer, cancer of the uterus, or melanoma.

10. Viral particle for use, according to any of claims 8 or 9, wherein the genotoxic chemotherapy drug is selected from the group comprising: cisplatin, hydroxyurea, 5-fluoroacyl, gemcitabine or cytosine arabinoside.

11. A pharmaceutical composition comprising a viral particle which in turn comprises a nucleotide sequence consisting essentially of: SEQ ID NO: 1, SEQ ID NO: 2, SEQ ID NO: 3 or SEQ ID NO: 4 in combination with a genotoxic chemotherapy drug.

12. Pharmaceutical composition according to claim 11, wherein the genotoxic chemotherapy drug is selected from the group comprising: cisplatin, hydroxyurea, 5-fluoruracyl, gemcitabine or cytosine arabinoside.

13. Method for determination of mutations in the TP53 gene and/or post-translational modifications in the p53 protein which comprises the use of a viral particle comprising a nucleotide sequence consisting essentially of: SEQ ID NO: 1, SEQ ID NO : 2, SEQ ID NO: 3 or SEQ ID NO: 4.

14. Method for *in vitro* diagnosis of cancer, or for selection of cancer patients, comprising the determination of mutations in the gene *TP53* and/or post-translational modifications in p53 protein using the viral particle comprising a nucleotide sequence essentially consisting of: SEQ ID NO: 1, SEQ ID NO: 2, SEQ ID NO: 3 or SEQ ID NO: 4.

15. Mutations in the TP53 gene, and/or post-translational modifications in the p53 protein for use in the treatment of cancer, where the mutations of the TP53 gene is selected from the group comprising: R273H, P72R, E258K, G245S or V173 L, and the post-translational modification of the p53 protein consists of phosphorylation at the Ser15 residue.


SEQUENCE LISTING

<110> UNIVERSIDAD AUTÓNOMA DE MADRID

<120> TRATAMIENTO CON PARVOVIRUS DE CÁNCER CON MUTACIONES EN EL GEN TP53
Y/O MODIFICACIONES POST-TRADUCCIONALES EN LA PROTEÍNA P53

<130> 903323

<160> 20

<170> PatentIn version 3.5

<210> 1
<211> 5149
<212> DNA
<213> Parvovirus diminuto de ratón MVMp

<400> 1
atttttagaa ctgaccaacc atgttcacgt aagtgacgtg atgacgcgcg ctgcgcgcgc 60

gccttcggac gtcacacgtc acttacgttt cacatggttg gtcagttcta aaaatgataa 120

gcggttcagg gagtttaaac caaggcgcga aaaggaagtg ggcgtggttt aaagtatata 180

agcaactact gaagtcagtt acttatcttt tctttcattc tgtgagtcga gacgcacaga 240

aagagagtaa ccaactaacc atggctggaa atgcttactc tgatgaagtt ttgggagcaa 300

ccaactggtt aaaggaaaaa agtaaccagg aagtgttctc atttgttttt aaaaatgaaa 360

atgttcaact gaatggaaaa gatatcggat ggaatagtta caaaaaagag ctgcaggagg 420

acgagctgaa atctttacaa cgaggagcgg aaactacttg ggaccaaagc gaggacatgg 480

aatgggaaac cacagtggat gaaatgacca aaaagcaagt attcattttt gattctttgg 540

ttaaaaaatg tttatttgaa gtgcttaaca caaagaatat atttcctggt gatgttaatt 600

ggtttgtgca acatgaatgg ggaaaagacc aaggctggca ctgccatgta ctaattggag 660

gaaaggactt tagtcaagct caaggaaaat ggtggagaag gcaactaaat gtttactgga 720

gcagatggtt ggtaacagcc tgtaatgtgc aactaacacc agctgaaaga attaaactaa 780

gagaaatagc agaagacaat gagtgggtta ctctacttac ttataagcat aagcaaacca 840

aaaaagacta taccaagtgt gttctttttg gaaacatgat tgcttactat tttttaacta 900

aaaagaaaat aagcactagt ccaccaagag acggaggcta ttttcttagc agtgactctg 960

gctggaaaac taacttttta aaagaaggcg agcgccatct agtgagcaaa ctatacactg 1020

atgacatgcg gccagaaacg gttgaaacca cagtaaccac tgcgcaggaa actaagcgcg 1080

gcagaattca aactaaaaaa gaagtttcta ttaaaactac acttaaagag ctggtgcata 1140

aaagagtaac ctcaccagag gactggatga tgatgcagcc agacagttac attgaaatga 1200

tggctcaacc aggtggagaa aacctgctga aaaatacgct agagatttgt acactaactc 1260

tagccagaac caaaacagca tttgacttaa ttttagaaaa agctgaaacc agcaaactaa 1320

ccaacttttc actgcctgac acaagaacct gcagaatttt tgcttttcat ggctggaact 1380

atgttaaagt ttgccatgct atttgctgtg ttttaaacag acaaggaggc aaaagaaata 1440

ctgttttatt tcatggacca gccagcacag gcaaatctat tattgcacaa gccatagcac 1500

aagcagttgg caatgttggt tgctataatg cagccaatgt aaactttcca tttaatgact 1560

gtaccaacaa gaacttgatt tgggtagaag aagctggtaa ctttggacag caagtaaacc 1620

agtttaaagc catttgctct ggtcaaacta ttcgcattga tcaaaaagga aaaggcagca 1680

aacagattga accaacacca gtcatcatga ccacaaatga gaacattaca gtggtcagaa 1740

taggctgcga agaaagacca gaacacactc aaccaatcag agacagaatg cttaacattc 1800

atctaacaca taccttgcct ggtgactttg gtttggttga caaaaatgaa tggcccatga 1860

tttgtgcttg gttggtaaag aatggttacc aatctaccat ggcaagctac tgtgctaaat 1920

ggggcaaagt tcctgattgg tcagaaaact gggcggagcc aaaggtgcca actcctataa 1980

atttactagg ttcggcacgc tcaccattca cgacaccgaa aagtacgcct ctcagccaga 2040

actatgcact aactccactt gcatcggatc tcgaggacct ggctttagag ccttggagca 2100

caccaaatac tcctgttgcg ggcactgcag aaacccagaa cactggggaa gctggttcca 2160

aagcctgcca agatggtcaa ctgagcccaa cttggtcaga gatcgaggag gatttgagag 2220

cgtgcttcgg tgcggaaccg ttgaagaaag acttcagcga gccgctgaac ttggactaag 2280

gtacgatggc gcctccagct aaaagagcta aaagaggtaa gggtttaagg gatggttggt 2340

tggtggggta ttaatgttta attacctgtt ttacaggcct gaaatcactt ggttttaggt 2400

tgggtgcctc ctggctacaa gtacctggga ccagggaaca gccttgacca aggagaacca 2460

accaatccat ctgacgccgc tgccaaagag cacgacgagg cctatgatca atacatcaaa 2520

tctggaaaaa atccttacct gtacttctct gctgctgatc aacgctttat tgaccaaacc 2580

aaggacgcca aagactgggg aggcaaggtt ggtcactact tttttagaac caagcgcgct 2640

tttgcaccta agcttgctac tgactctgaa cctggaactt ctggtgtaag cagagctggt 2700

aaacgcacta gaccacctgc ttacattttt attaaccaag ccagagctaa aaaaaaactt 2760

acttcttctg ctgcacagca aagcagtcaa accatgagtg atggcaccag ccaacctgac 2820

agcggaaacg ctgtccactc agctgcaaga gttgaacgag cagctgacgg ccctggaggc 2880

tctgggggtg ggggctctgg cgggggtggg gttggtgttt ctactgggtc ttatgataat 2940

caaacgcatt atagattctt gggtgacggc tgggtagaaa ttactgcact agcaactaga 3000

ctagtacatt taaacatgcc taaatcagaa aactattgca gaatcagagt tcacaataca 3060

acagacacat cagtcaaagg caacatggca aaagatgatg ctcatgagca aatttggaca 3120

ccatggagct tggtggatgc taatgcttgg ggagtttggc tccagccaag tgactggcaa 3180

tacatttgca acaccatgag ccagcttaac ttggtatcac ttgatcaaga aatattcaat 3240

gtagtgctga aaactgttac agagcaagac ttaggaggtc aagctataaa aatatacaac 3300

aatgacctta cagcttgcat gatggttgca gtagactcaa acaacatttt gccatacaca 3360

cctgcagcaa actcaatgga aacacttggt ttctacccct ggaaaccaac catagcatca 3420

ccatacaggt actatttttg cgttgacaga gatctttcag tgacctacga aaatcaagaa 3480

ggcacagttg aacataatgt gatgggaaca ccaaaaggaa tgaattctca attttttacc 3540

attgagaaca cacaacaaat cacattgctc agaacagggg acgaatttgc cacaggtact 3600

tactactttg acacaaattc agttaaactc acacacacgt ggcaaaccaa ccgtcaactt 3660

ggacagcctc cactgctgtc aacctttcct gaagctgaca ctgatgcagg tacacttact 3720

gctcaaggga gcagacatgg aacaacacaa atgggggtta actgggtgag tgaagcaatc 3780

agaaccagac ctgctcaagt aggattttgt caaccacaca atgactttga agccagcaga 3840

gctggaccat ttgctgcccc aaaagttcca gcagatatta ctcaaggagt agacaaagaa 3900

gccaatggca gtgttagata cagttatggc aaacagcatg gtgaaaattg ggcttcacat 3960

ggaccagcac cagagcgcta cacatgggat gaaacaagct ttggttcagg tagagacacc 4020

aaagatggtt ttattcaatc agcaccacta gttgttccac caccactaaa tggcattctt 4080

acaaatgcaa accctattgg gactaaaaat gacattcatt tttcaaatgt ttttaacagc 4140

tatggtccac taactgcatt ttcacaccca agtcctgtat accctcaagg acaaatatgg 4200

gacaaagaac tagatcttga acacaaacct agacttcaca taactgctcc atttgtttgt 4260

aaaaacaatg cacctggaca aatgttggtt agattaggac caaacctaac tgaccaatat 4320

gatccaaacg gagccacact ttctagaatt gttacatacg gtacattttt ctggaaagga 4380

aaactaacca tgagagcaaa acttagagct aacaccactt ggaacccagt gtaccaagta 4440

agtgctgaag acaatggcaa ctcatacatg agtgtaacta aatggttacc aactgctact 4500

ggaaacatgc agtctgtgcc gcttataaca agacctgttg ctagaaatac ttactaacta 4560

accatgcttt ttctttctgt acttcatata ttattaagac taataaagat acaacataga 4620

aatataatat tacgtataga tttaagaaat agaataatat ggtacttagt aactgttaaa 4680

aataatagaa cctttggaat aacaagatag ttagttggtt aatgttagat agaataagaa 4740

gatcatgtat aatgaataaa agggtggaag ggtggttggt aggttaatgt tagatagaat 4800

aagaagatca tgtataatga ataaaagggt ggaagggtgg ttggtaggta ttcccttaga 4860

cttgatgtta aggaccaaaa aaataataaa acttttttaa aactcaacca agactactgt 4920

ctattcagtg aaccaactga accattagta ttactatgtt tttagggtgg gagggtggga 4980

gatacatgtg ttcgctatga gcgaactggt actggttggt tgctctgctc aaccaaccag 5040

accggcaaag ccggtctggt tggttgagcg caaccaacca gtaccagttc gctcatagcg 5100

aacacatgta tctcccaccc tcccacccta aaaacatagt aatactaat 5149

<210> 2
<211> 5087
<212> DNA
<213> Parvovirus diminuto de ratón MVMi

<400> 2
atttttagaa ctgaccaacc atgttcacgt aagtgacgtg atgacgcgcg cttcgcgcgc 60

tgcctgcggc agtcacacgt cacttacgtc tcacatggtt ggtcagttct aaaaatgata 120

agcggttcag agagtttaga ccaaggcgcg aaaaggaagt gggcgtggtt taaagtatat 180

aagcaaatgc tgaagtcagt tacttatcct ttctttcatt ctgtgagtcg agacgcgcag 240

aaagagagta accaactaac catggctgga aatgcttact ctgatgaagt tttgggaaca 300

accaactggt taaaggaaaa aagtaaccag gaagtgttct catttgtttt taaaactgag 360

gatgttcaac taaatggaaa agatatcgga tggaataatt acaaaaagga gctgcaggag 420

gacgagctga aatctttaca acgaggagcg gaaactacct gggaccaaag cgaggacatg 480

gaatgggaat ctacagtgga tgaaatgacc aaaaagcaag tattcattta tgactcttta 540

gttaaaaaat gtttgtttga agtgcttagc acaaaaaata tagctcctgc tgatgttact 600

tggtttgtgc agcatgaatg ggggaaagac caaggctggc actgccatgt actaattgga 660

ggcaaggact ttagtcaagc tcaaggaaaa tggtggagaa ggcagctaaa tgtttactgg 720

agcagatggt tggtaacagc ctgtaatgtg cagctaacac cagctgaaag aattaaacta 780

agagaaatag cagaagacag tgagtgggtt actttactca cttataaaca taagcaaacc 840

aaaaaggact atactaaatg tgttcttttt ggaaatatga ttgcttacta ctttttaacc 900

aaaaagaaaa taagcaccag tccgccaagg gacggaggct attttctaag cagtgactct 960

ggctggaaaa ctaacttttt aaaagagggc gaacgccatc tagtgagcaa attatacact 1020

gatgacatgc ggccagaaac ggttgaaacc acagtaacca ctgcgcagga aactaagcgc 1080

ggcagaattc aaactaaaaa agaggtttct attaaaacca cacttaaaga gctagtgcat 1140

aaaagagtaa cctcaccaga agactggatg atgatgcagc cagacagtta cattgaaatg 1200

atggctcaac caggtggaga aaacctgctg aaaaatacgc tagagatttg tacgctaact 1260

ctagccagaa caaaaacagc atttgacttg attttagaaa aagctgaaac cagcaaacta 1320

accaactttt cactgcctga cacaagaacc tgcaagattt ttgcttttca tggctggaac 1380

tatgttaaag tttgccatgc tatttgctgt gttctaaaca gacaaggagg caaaagaaat 1440

actgttttat ttcacggacc agccagtaca ggcaaatcta ttattgcaca agccatagca 1500

caggcagttg gtaatgttgg ttgctataat gcagctaatg tgaactttcc atttaatgac 1560

tgtacgaaca aaaacttgat ttgggtagaa gaagctggta actttggaca gcaagtaaac 1620

cagtttaaag ccatttgctc tggtcaaact attcgcattg atcaaaaagg aaaaggcagc 1680

aaacaaattg aaccaacacc agtcatcatg accacaaatg agaacattac agtggtcaga 1740

ataggctgcg aagagagacc agaacacact caaccaatta gagacagaat gctcaacatt 1800

catctaacac atacattgcc tggtgacttt ggtttggttg acaagaatga atggcccatg 1860

atttgtgctt ggttggtaaa gaatggttac caatctacca tggcaagcta ctgcgctaaa 1920

tggggcaaag ttcctgattg gtcagaaaac tgggcggagc cgaaggtgcc gactcctata 1980

aattcactag gttcggcacg ctcaccattc acgacaccga aaagtacgcc tctcagccag 2040

aactatgcac taactccact tgcatcggat ctcgaggacc tggctttaga gccttggagc 2100

acaccaaata ctcctgttgc gggcactgca gaaacccaga acactgggga agctggttcc 2160

aaagcctgcc aagatggtca actgagccca acttggtcag agatcgagga ggatttgaga 2220

gcgtgcttcg gtgcggaacc gttgaagaga gacttcagcg agccgctgaa cttggactaa 2280

ggtacgatgg cgcctccagc taaaagagct aaaagaggta agggtttaag ggatggttgg 2340

ttggtggggt attaatgttt aattacctgt tttacaggcc tgaaatcact tggttttagg 2400

ttgggtgcct cctggctata agtacctggg accagggaac agccttgacc aaggagaacc 2460

aaccaatcca tctgacgccg ctgccaaaga gcacgacgag gcctatgatc aatacatcaa 2520

atctggaaaa aatccttacc tgtacttctc tgctgctgat caacgcttta ttgaccaaac 2580

caaggacgcc aaagactggg gaggcaaggt tggtcactac ttttttagaa ccaagcgcgc 2640

ttttgcacct aagcttgcta ctgactctga acctggaact tctggtgtaa gcagagctgg 2700

taaacgcact agaccacctg cttacatttt tataaaccaa gccagagcta aaaaaaaact 2760

tacttcttct gctgcacagc aaagcagtca aaccatgagt gatggcacca gccaacctga 2820

cggcggaaac ggtgtccact cagctgcaag agttgaacga gcagctgacg gccctggagg 2880

ctctgggggt gggggctctg gcgggggtgg ggttggtgtt tctactgggt cttatgataa 2940

tcagacgcat tatagattct tgggtgacgg ctgggtagaa attactgcac tagcaactag 3000

actagtacat ttaaacatgc ctaaatcaga aaactattgc agaataagag ttcacaacac 3060

aacagacact tcagtcaaag gcaacatggc aaaagatgat gctcatgagc aaatttggac 3120

gccatggagc ttagtggatg ctaatgcttg gggagtttgg ctccagccaa gtgactggca 3180

atacatttgc aacaccatga gccagcttaa cttggtctca cttgatcaag aaatatttaa 3240

tgtagtgctg aaaactgtta cagagcaaga ctcaggaggt caagctataa aaatatacaa 3300

caatgacctc acagcttgca tgatggttgc agtagactca aacaacattc tgccatacac 3360

acctgcagca aactcaatgg aaacacttgg tttctaccct tggaaaccaa ctatagcatc 3420

gccatacagg tactatttct gcgttgacag agatctttca gtaacctatg aaaatcaaga 3480

aggcacaatt gagcataatg taatgggaac accaaaagga atgaattctc aattttttac 3540

cattgagaac acacaacaaa tcacattgct cagaactggt gatgagtttg ctactggaac 3600

ctactacttt gacacaaacc cagttaaact tacacacaca tggcaaacta accgtcaact 3660

tggacagcct ccactgctgt caacctttcc tgaagctgac actgatgcag gtacacttac 3720

tgctcaaggg agcagacatg gagcaacaca gatggaggtt aactgggtga gtgaagcaat 3780

tagaaccaga cctgctcaag taggattttg tcagccacac aatgactttg aagccagcag 3840

agctggacca tttgctgctc caaaagttcc agcagatgtt actcaaggag tggacagaga 3900

agccaatggc agtgttagat acagttatgg caaacagcat ggtgaaaatt gggctgcaca 3960

cggaccagca ccagagcgct acacatggga tgaaacaaac tttggttcag gaagagacac 4020

cagagatggt tttattcaat cagcacctct agttgttcca ccaccactaa atgggattct 4080

tacaaatgca aaccctattg gaactaaaaa tgacattcat ttttcaaatg tttttaacag 4140

ctatggtcca ctaactgcat tttcacaccc aagtcctgta taccctcaag gacaaatatg 4200

ggacaaagaa ctagatcttg aacacaaacc tagacttcac ataactgctc catttgtctg 4260

taaaaacaat gcacctggac aaatgttggt tagattagga ccaaatctaa ctgaccagta 4320

tgatccaaac ggagccacac tttctagaat tgtgacttat ggtacatttt tctggaaagg 4380

aaaactaaca atgagagcaa aacttagagc taacaccact tggaacccag tgtaccaagt 4440

aagtgttgaa gacaatggca actcatacat gagtgttact aaatggctac caactgctac 4500

tggaaacatg caatctgtac cgcttataac aagacctgtt gctagaaata cttactaact 4560

aaccatgttt ttcctttctg tacttcatat attattaaga ctaataaaga tacaacataa 4620

aaatataata ttacatatag atttaagaaa tagaataata tggtacttag taactgttag 4680

aaataataga acctttggaa taacaagata attagttggt taatgttaga tagaataaga 4740

agattatgta taatgggtaa aagggtggaa gggtggttgg ttggtattcc cttagacatg 4800

atgttaagga ccaaaaaaat aataaaattt tttaaaacta aaccaagact actgtctatt 4860

cagttgaacc aactgaacca tcagtatcac tatgttttta gggtgggggg gtgggagata 4920

catgtgttcg ctatgagcga actggtactg gttggttgct ctgctcaacc aaccagaccg 4980

gctttgccgg tctggttggt tgagcgcaac caaccagtac cagttcgctc atagcgaaca 5040

catgtatctc ccaccccccc accctaaaaa catagtgata ctgatgg 5087

<210> 3
<211> 5135
<212> DNA
<213> Parvovirus LuIII

<400> 3
atcattttta gaactaacca accatgttca cgtaagtgac gtgatgacgc gcgctacgcg 60

cgctgccttc ggcagtcaca cgtcacttac gtctcacatg gttggttagt tctaaaaatg 120

ataagcggtt cagggagttt aaaccaaggc gcgaaaagga agtgggcgtg gttttaagta 180

tataagcgac acgttaagtc agttacttac tctttcgctt attctgtaag tcgagacaca 240

cagagtaacc aactaaccaa ctagccatgg ctggaaacgc gtactctgat gaagttttgg 300

gaacaactaa ctggttgaag gataagagca accaggaagt attctcattt gtttttaaaa 360

atgaggatgt tcagctcaat ggaaaaaata tcggatggaa cagttacaga aaggagctgc 420

aagaggagga gctgaaatct ttacaacgag gagctgaaac tacctgggac cagagcgagg 480

acatggaatg ggaatcttca gtggatgaac tgaccaaaaa gcaagtattc atttttgact 540

ctttagttaa aaagtgtctc tttgaagtac tgagcacaaa gaacatagct cctagtgatg 600

ttacttggtt tgtacagcat gaatggggaa aagaccaagg ctggcactgt catgtgctca 660

ttggaggcaa gaactttagc caggctcaag gaaaatggtg gaggagacaa ttaaatgttt 720

actggagtag atggttggta acagcctgta gcgtgcagct atcaccagct gaaagaatta 780

aactaagaga aatagcagaa gaccaagaat gggttactct gcttacttat aagcataagc 840

aaaccaaaaa agactatact aagtgtgttt gctttggaaa tatggttgct tactactttt 900

taaccaaaaa gaaaatatgt accagtccac caagggacgg aggctatttt ctcagtagtg 960

actctggctg gaaaactaac tttttgaaag aaggcgaacg ccatctagtg agcaaactat 1020

atactgatga catgcggcca gaaacggttg agaccacagt aaccacagcg caggaaacta 1080

agcgcggcag aattcaaact aagaaggaag tctctattaa gactacactt aaagagctgg 1140

tacataagag agtaacctca ccagaagact ggatgatgat gcagccagac agttacattg 1200

aaatgatggc tcaaccaggg ggagaaaacc tacttaagaa tacgctagag atctgtacgc 1260

tgactctagc cagaaccaaa acagcctttg acttgatttt agaaaaagct gaaaccagca 1320

aactaaccaa ctttttactg gctgatacaa gaacctgtag aatctttgct tttcatggct 1380

ggaactacat caaagtctgt catgctattt gttgtgtctt gaacagacag ggaggcaaaa 1440

gaaatactgt tctgtttcat ggaccagcca gtacaggcaa atcaatcatt gcacaggcca 1500

tagcacaggc agttggtaat gttggttgtt ataacgcagc caatgtgaac tttccattta 1560

atgactgtac caacaagaac ttaatctggg tggaagaagc tggtaacttt ggacagcaag 1620

taaaccagtt taaagccatt tgttctggtc agaccattcg cattgaccaa aaaggaaaag 1680

gcagcaaaca gattgaacca acaccagtga tcatgaccac aaatgaaaac atcacagtgg 1740

tcaaaatagg gtgtgaagag agaccagaac acactcaacc aatcagagac agaatgttaa 1800

acattcatct gacacataca ttgcctggtg actttggttt ggttgataaa aacgaatggc 1860

ctatgatatg tgcttggttg gtaaagaacg gttaccaatc gaccatggca agttactgtg 1920

ctaaatgggg caaagttcct gattggacag aaaactgggc ggagccaaaa gtaacgactg 1980

aaataaattc ggtaggttca accaactcac catctccgaa aagtacgcct ctcagccaga 2040

actacgcact aactccgtcg gatctcgagg acctggctct ggagccttgg agcacaccaa 2100

gtactcctgt tgtgggcact gtcaaaaccc cgaacactgg ggaaactggt tcaacagcct 2160

gtcaagaagc tcaacggagc ccaacttggt ccgagatcga ggaggatttg agagcgtgct 2220

tcagttcgga acactggaag agcgactccg aacagctacc aaacttggat taaggtacga 2280

tggcgcctcc ggctaaaaga gctaaaagag gtaaggggtt aagggatggt tggtaggttg 2340

gtggggtatt aatatgtgac tacctgtttt acaggcctga aatcacttgg ttctaggttg 2400

ggtgcctcca ggctacaagt acctgggacc agggaacagc cttaaccaag gagaaccaac 2460

caatccatct gacgctgctg ctaaagagca cgacgaggcc tacgaccaat acatcaaatc 2520

tggaaagaat ccttacctgt acttctctcc tgctgatcaa cgcttcattg accaaaccaa 2580

agacgctaaa gactggggcg gaaaggttgg tcactacttc tttagaacca agcgtgcttt 2640

tgcacctaag ctttctactg actctgagcc tgggacttct ggtgtgagca cagctggtaa 2700

acgtactaaa ccacctgctc acatctttat taaccaagcc agggctaaaa aaaaacgtac 2760

ttctcttgct gcgcagcaga ggactcagac aatgagtgat ggcaccgacc aatctgacag 2820

cggaaacgct gtccagtcag ctgctagagt tgagcgagca gctgacggtc ctggaggctc 2880

tgggggcggg ggctctggtg ggggtggggt tggcgtttct actggcagtt atgataatca 2940

aacacattat aagtttctag gggatgggtg ggtagagatt actgcttaca gcacacgcat 3000

ggtacacttg aacatgccta aatcagaaaa ctactgtagg gtgcgcgtac acaacacaaa 3060

tgacacaggt acagcaagtc acatggctat ggacgatgct catgaacaga tttggacacc 3120

atggagtctg gttgatgcta atgcttgggg agtttggttt caaccaagtg actggcagta 3180

catttctaat aatatgattc acatcaattt acattcactt gaccaagaat tgtttaatgt 3240

ggtcatcaaa acagtgactg aacagaacac aggagctgag gccattaagg tctacaacaa 3300

tgacctcact gctgccatga tggttgctct tgattctaac aacatactgc cttacacacc 3360

agccatagac aatcaagaga cacttggttt ctatccatgg aaaccaacca taccaagtcc 3420

ttacagatac tattttagct gtgacagaaa cttatcagtt acttacaaag acgaagcagg 3480

aaccatcact gacacaatgg gtttggccag tggcctgaac tcccaatttt ttaccattga 3540

gaacactcag cgtattaacc tactcagaac tggggatgag tatgctactg gaacttacta 3600

ctttgacaca gaaccaatca gactaactca cacgtggcaa accaacagac acctgggtca 3660

gcctccacaa attactgaac taccaagctc tgacactgct aacgctactt taacagctag 3720

aggttacaga tcaggtctga ctcaaattca aggcagaaat gatgtgactg aagctactag 3780

ggtcagacct gcacaggttg gattttgtca gcctcatgac aattttgaaa ccagcagagc 3840

ggggcctttc aaggttccgg tagtgccagc agacatcaca caaggcctag accatgatgc 3900

caatggtagc ctgagatata cctatgacaa acaacatggt caaagctggg caagtcagaa 3960

caacaaagac aggtacactt gggatgctgt taactatgat tctggcagat ggactaacaa 4020

ctgttttatt caatcagtac catttacatc agaaccaaat gctaaccaaa tacttactaa 4080

ccgtgacaac ctagcgggta agactgacat acattttacc aacgcattta acagttatgg 4140

accactaact gcttttccac atcctgcgcc gatttaccca caagggcaga tttgggacaa 4200

agaacttgat cttgaacaca agccaagact gcacacacag gctccttttg tctgtaaaaa 4260

caatgctcca ggtcagcttc tggttaggct agcacctaac ttgactgacc agtatgatcc 4320

taatagttct aacctatcta gaattgtcac ctatggcacc ttcttctgga agggcaaact 4380

aactctaaaa gcaaagatga gacctaatgc tacttggaac ccagtcttcc aaataagtgc 4440

taccaaccaa ggaaccaatg actacatgag cattgaaaga tggttaccaa ctgctactgg 4500

caacataaca aatgtgcctc tgctttctag acctgttgct agaaacactt actaactaac 4560

tatgctctat gcttcatata tattatatat attattatac taactaacca tgtttactct 4620

tacattactt catataatat taagactaat aaaaatacaa catagaaata taatattaca 4680

tatagatata aagaatagaa taatatggta cttacttact gttagaaata atagaacttt 4740

tggaataaca agatagttag ttggtttatg ttatatagaa tataagaaga tgatgtacaa 4800

agaataaaag ggtgggaggg tggttggttg gtactccctt agactgaatg ttagggacca 4860

aaaaaataat aaaattcttg aaaacccaac aaggactact gtcatattca gtgaaccaac 4920

tgaaccatta gtatcaatat gattttaggg tgggggggtg ggagatacat atgttcacta 4980

tggaccaact ggtactggtt ggttgctctg ctccaaccaa ccagaccggc tctgccggtc 5040

tggttggttg agcgcaacca accagtacca gttggtccat agtgaacata tgtatctccc 5100

acccccccac cctaaaaaca tattgatact aatgg 5135

<210> 4
<211> 5176
<212> DNA
<213> Parvovirus H-1

<400> 4
catttttaga actgaccaac catgttcacg caagtgacgt gatgacgcgc gctgcgcgcg 60

ctgccttcgg cagtcacacg tcactagcgt ttcacatggt tggtcagttc taaaaatgat 120

aagcggttca gagagtttga aaccaaggcg ggaaacggaa gtgggcgtgg ctaactgtat 180

ataagcagtc actctggtcg gttactcact ctgctttcat ttctgagttt gtgagacaca 240

ggagcgagac taaccaacta accatggctg gaaacgctta ctccgatgag gttttgggag 300

taacaaactg gctgaaggac aaaagtagcc aggaggtgtt ctcatttgtt tttaaaaatg 360

aaaacgtcca actaaatgga aaggacatcg gttggaatag ttacagaaag gagctacaag 420

atgacgagct gaagtctcta caacgagggg cggagaccac ttgggaccaa agcgaggaca 480

tggaatggga gagcgcagtg gatgacatga ccaaaaagca agtatttatt tttgattctt 540

tggttaagaa gtgtttgttt gaagtgctca gcacaaagaa catagctcct agtaatgtta 600

cttggttcgt gcagcatgaa tggggaaagg acccaggctg gcactgtcat gtgctgattg 660

gaggcaagga ctttagtcaa cctcaaggaa aatggtggag aaggcagcta aatgtgtact 720

ggagtagatg gttggtgact gcctgtaatg ttcaactaac accagctgaa agaattaaac 780

tgagagaaat agcagaggac agtgaatggg tcactttgct tacctataag cataagcaca 840

ccaagaagga ctataccaag tgtgttcttt ttggaaacat gattgcttat tactttttaa 900

gcaaaaagaa aatatgtacc agtccaccaa gggacggagg ctattttctt agcagtgact 960

ctggctggaa aactaacttt ttgaaagagg gcgagcgcca tctagtgagc aaactgtata 1020

ctgatgagat gaaaccagaa acggtcgaga ccacagtgac cactgcacag gaagctaagc 1080

gcggcagaat tcaaactaga gaggaggtct cgattaaaac cacactcaaa gagttggtac 1140

ataaaagagt aacctcacca gaagactgga tgatgatgca gccagacagt tacattgaaa 1200

tgatggctca accaggtgga gaaaacttgc ttaaaaatac actagagatc tgtacactga 1260

ctctagcaag aaccaaaaca gcctttgact tgattctgga aaaagctgaa accagcaaac 1320

tagccaactt ttccatggct agcaccagaa cctgtagaat ctttgctgag catggctgga 1380

actatattaa agtctgccat gccatctgtt gtgtgctgaa tagacaagga ggcaaaagga 1440

acactgtgct ctttcacgga ccagccagca caggcaaatc tattattgca caagccatag 1500

cacaagcagt tggtaatgtt ggttgttaca atgctgccaa tgtgaacttt ccatttaatg 1560

actgtaccaa caaaaacttg atttgggtgg aagaagctgg taactttggc cagcaagtaa 1620

accaattcaa agctatttgt tctggccaaa ccatacgcat tgatcaaaaa ggaaaaggca 1680

gcaaacagat tgaaccaaca ccagttatta tgaccaccaa cgagaacatt accgtggtta 1740

gaataggctg tgaggaaaga ccagaacaca ctcaaccaat cagagacaga atgctcaaca 1800

ttcacctgac acgtacacta cctggtgact ttggtttggt ggataagcac gaatggcctc 1860

tgatctgtgc ttggttggtg aagaatggtt accaatctac catggcttgt tactgtgcta 1920

aatggggcaa agttcctgat tggtcagagg actggcggga gccgaagcta gacactccta 1980

taaattcgct aggttcaatg cgctcaccat ctctgactcc gagaagtacg cctctcagcc 2040

aaaactacgc tcttactcca cttgcatcgg accttgcgga cctagctcta gagccttgga 2100

gcacaccaaa tactcctgtt gcgggcactg cagcaagcca aaacactggg gaggctggtt 2160

ccacagcctg ccaaggtgct caacggagcc caacctggtc cgagatcgag gcggatttga 2220

gagcttgctt cagtcaagaa cagttggaga gcgacttcaa cgaggagctg accttggact 2280

aaggtacaat ggcacctcca gctaaaagag ctaaaagagg taaggggcta agggatggtt 2340

ggttggtggg gtactaatgt atgactacct gttttacagg cctgaaatca cttggttcta 2400

ggttgggtgc ctcctggcta caagtacctg ggaccaggga acagccttga ccaaggagaa 2460

ccaaccaacc cttctgacgc cgctgccaaa gaacacgacg aagcctacga ccaatacatc 2520

aaatctggaa aaaatcctta cctgtacttc tctcctgctg atcaacgctt cattgaccaa 2580

accaaagacg ccaaggactg gggcggcaag gttggtcact acttttttag aaccaagcga 2640

gcttttgcac ctaagctttc tactgactct gaacctggca cttctggtgt gagcagacct 2700

ggtaaacgaa ctaaaccacc tgctcacatt tttgtaaatc aagccagagc taaaaaaaaa 2760

cgcgcttctc ttgctgcaca gcagaggact ctgacaatga gtgatggcac cgaaacaaac 2820

caaccagaca ctggaatcgc taatgctaga gttgagcgat cagctgacgg aggtggaagc 2880

tctgggggtg ggggctctgg cgggggtggg attggtgttt ctactgggac ttatgataat 2940

caaacgactt ataagttttt gggagatgga tgggtagaaa taactgcaca tgcttctaga 3000

cttttgcact tgggaatgcc tccttcagaa aactactgcc gcgtcaccgt tcacaataat 3060

caaacaacag gacacggaac taaggtaaag ggaaacatgg cctatgacac acatcaacaa 3120

atttggacac catggagctt ggtagatgct aatgcttggg gagtttggtt ccaaccaagt 3180

gactggcagt tcattcaaaa cagcatggaa tcgctgaatc ttgactcatt gagccaagaa 3240

ctatttaatg tagtagtcaa aacagtcact gaacaacaag gagctggcca agatgccatt 3300

aaagtctata ataatgactt gacggcctgt atgatggttg ctctggatag taacaacata 3360

ctgccttaca cacctgcagc tcaaacatca gaaacacttg gtttctaccc atggaaacca 3420

accgcaccag ctccttacag atactacttt ttcatgccta gacaactcag tgtaacctct 3480

agcaactctg ctgaaggaac tcaaatcaca gacaccattg gagagccaca ggcactaaac 3540

tctcaatttt ttactattga gaacaccttg cctattactc tcctgcgcac aggtgatgag 3600

tttacaactg gcacctacat ctttaacact gacccactta aacttactca cacatggcaa 3660

accaacagac acttggcatg cctccaagga ataactgacc taccaacatc agatacagca 3720

acagcatcac taactgcaaa tggagacaga tttggatcaa cacaaacaca gaatgtgaac 3780

tatgtcacag aggctttgcg caccaggcct gctcagattg gcttcatgca acctcatgac 3840

aactttgaag caaacagagg tggcccattt aaggttccag tggtaccgct agacataaca 3900

gctggcgagg accatgatgc aaacggagcc atacgattta actatggcaa acaacatggc 3960

gaagattggg ccaaacaagg agcagcacca gaaaggtaca catgggatgc aattgatagt 4020

gcagctggga gggacacagc tagatgcttt gtacaaagtg caccaatatc tattccacca 4080

aaccaaaacc agatcttgca gcgagaagac gccatagctg gcagaactaa catgcattat 4140

actaatgttt ttaacagcta tggtccactt agtgcatttc ctcatccaga tcccatttat 4200

ccaaatggac aaatttggga caaagaattg gacctggaac acaaacctag actacacgta 4260

actgcaccat ttgtttgtaa aaacaaccca ccaggtcaac tatttgttca cttggggcct 4320

aatctgactg accaatttga cccaaacagc acaactgttt ctcgcattgt tacatatagc 4380

actttttact ggaagggtat tttgaaattc aaagccaaac taagaccaaa tctgacctgg 4440

aatcctgtat accaagcaac cacagactct gttgccaatt cttacatgaa tgttaagaaa 4500

tggctcccat ctgcaactgg caacatgcac tctgatccat tgatttgtag acctgtgcct 4560

cacatgacat actaaccaac caactatgtt tctctgtttg cttcacataa tacttaaact 4620

aactagacta caacataaaa atatacactt aataatagat tattaaaaat aacataatat 4680

ggtaggttaa ctgtaaaaaa taatagaact tttggaataa atatagttag ttggttaatg 4740

ttagatagaa tataaaaaga ttttgtattt taaaataaat atagttagtt ggttaatgtt 4800

agatagaata taaaaagatt ttgtatttgg gaaataaaaa gggtggttgg gtggttggtt 4860

ggtactccct tagactgaat gttagggacc aaaaaaataa taaaataatt aaaatgaaca 4920

aggactactg tctattcagt tgaccaactg aacctatagt atcactatgt ttttagggtg 4980

ggggggtggg agatacatac gttcgctatg gaccaagtgg taccggttgg ttgctaagct 5040

cgaacaagac ggctaagccg gtccggttgg ttgagcgcaa ccaaccggta ccacttggtc 5100

catagcgaac gtatgtatct cccacccccc caccctaaaa acatagtgat actataggtt 5160

cagttggtca actgaa 5176

<210> 5
<211> 21
<212> DNA
<213> GS7 (+/+)

<400> 5
gctgctcccc ccgtggcccc t 21

<210> 6
<211> 21
<212> DNA
<213> GS7 (+/-)

<400> 6
catcacactg gaagactcca g 21

<210> 7
<211> 21
<212> DNA
<213> GS5 (+/+)

<400>  7
ctgcatgggc ggcatgaacc g                                                21

<210>  8
<211>  21
<212>  DNA
<213>  GS5 (+/-)

<400>  8
cagggctcac tccagccacc t                                                21

<210>  9
<211>  15
<212>  DNA
<213>  TP53, NIH3T3, MEF

<400>  9
ggaggtcgtg agacg                                                       15

<210>  10
<211>  15
<212>  DNA
<213>  A9, A9(+/+), A9(+/-)

<400>  10
ggaggtcctg agacg                                                       15

<210>  11
<211>  15
<212>  DNA
<213>  TP53, GS5, GS5(+/+), GS5(+/-),GS7 (+/+)

<400>  11
catgggcggc atgaa                                                       15

<210>  12
<211>  15
<212>  DNA
<213>  GS8, GS9

<400>  12
catgggcagc atgaa                                                       15

<210>  13
<211>  18

<212>  DNA
<213>  Artificial sequence

<220>
<223>  Primer forward 34-421

<400>  13
gcagtcagat cctagcgt                                                    18


<210>  14
<211>  18
<212>  DNA
<213>  Artificial sequence

<220>
<223>  Primer reverse 34-421

<400>  14
catcttgttg agggcagg                                                    18


<210>  15
<211>  19
<212>  DNA
<213>  Artificial sequence

<220>
<223>  Primer forward 301-654

<400>  15
gtcatcttct gtcccttcc                                                   19


<210>  16
<211>  24
<212>  DNA
<213>  Artificial sequence

<220>
<223>  Primer reverse 301-654

<400>  16
gtgtttctgt catccaaata ctcc                                             24


<210>  17
<211>  20
<212>  DNA
<213>  Artificial sequence

<220>
<223>  Primer forward 609-990

<400>  17
gagtggaagg aaatttgcgt                                                  20


<210>  18
<211>  21
<212>  DNA
<213>  Artificial sequence

<220>
<223>  Primer reverse 609-990

<400>  18
agtggtttct tctttggctg g                                                21


<210>  19
<211>  19
<212>  DNA
<213>  Artificial sequence

<220>
<223>  Primer forward 948-1225

<400>  19
ccaacaacac cagctcctc                                                   19


<210>  20
<211>  24
<212>  DNA
<213>  Artificial sequence

<220>
<223>  Primer reverse 948-1225

<400>  20
ggaacaagaa gtggagaatg tcag                                             24